(19) Europäisches Patentamt
European Patent Office
Office européen des brevets

(11) EP 2 444 107 A1

(12) **EUROPEAN PATENT APPLICATION**
published in accordance with Art. 153(4) EPC

(43) Date of publication:
**25.04.2012 Bulletin 2012/17**

(21) Application number: **10789573.2**

(22) Date of filing: **18.06.2010**

(51) Int Cl.:
***A61L 9/01*** (2006.01) ***A61L 9/14*** (2006.01)
***A62D 3/30*** (2007.01) ***C02F 1/30*** (2006.01)
***C02F 1/68*** (2006.01) ***C09K 3/00*** (2006.01)
***A62D 101/20*** (2007.01)

(86) International application number:
**PCT/JP2010/060333**

(87) International publication number:
**WO 2010/147200 (23.12.2010 Gazette 2010/51)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **18.06.2009 JP 2009145609**

(71) Applicants:
- **Orbs Co., Ltd.**
**Nagano 399-9301 (JP)**
- **Nozaki, Atsuo**
**Koriyama-shi, Fukushima 963-8041 (JP)**

(72) Inventor: **KISHI Kiyomi**
**Kitaazumi-gun**
**Nagano 399-9301 (JP)**

(74) Representative: **Vossius & Partner**
**Siebertstrasse 4**
**81675 München (DE)**

(54) **CONTAINMINANT TREATMENT AGENT FOR LIVING SPACES, AND METHODS FOR MANUFACTURING AND USING SAME**

(57) In order to suppress emission of contaminants (such as volatile organic compounds contained in building materials and furniture) in living spaces into the outside and to provide a deodorant effect, provided is a contaminant treatment agent tobe applied to the surfaces of contaminant sources (such as building materials and furniture (2)) in a living space such as a room space in a building (1), to thereby treat contaminants (such as volatile organic compounds (3)) contained in the building materials and furniture (2), the contaminant treatment agent including: stable pure water (4) which is excellent in oil component solubility and has a smaller time-dependent change in absorbance measured with a spectrophotometer under the condition of being left to stand for at least 5 hours or more than pure water including purified water, the stable pure water (4) being prepared by irradiating pure water including neutral water free of the occurrence of an electric charge imbalance with light collected by causing natural light to pass through a light collector using titanium or silicon; eucalyptus essential oil (5) that is dissolved in the stable pure water (4) and contains cineol as a main component; and a pre-selected mineral component that is added to the stable pure water (4).

FIG.1

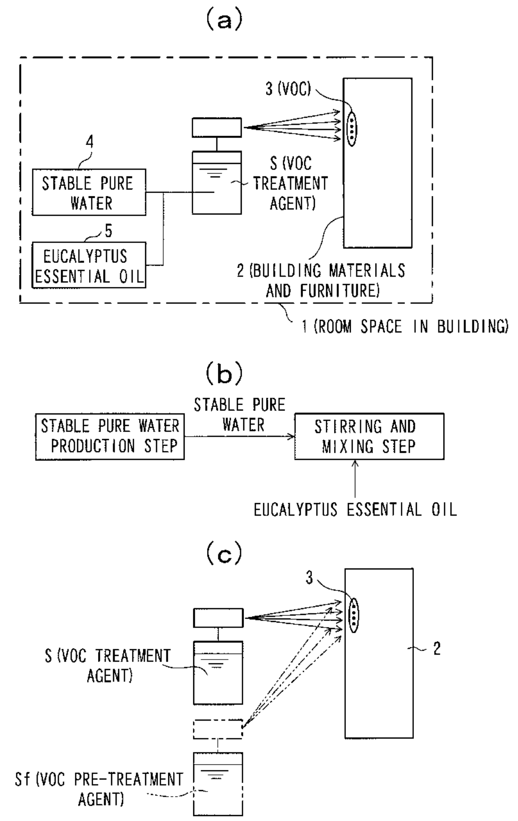


EP 2 444 107 A1

## Description

### Technical Field

The present invention relates to a treatment agent for treating contaminants in living spaces, for example, volatile organic compounds such as formaldehyde emitted from building materials and furniture, a method of producing the treatment agent, and a method of using the treatment agent.

### Background Art

In recent years, living environment pollution has been attracting attention as a big problem. The pollution affecting human bodies most directly is, for example, air pollution caused by volatile organic compounds (VOCs) generated from building materials and furniture in room spaces in a building (such as residential spaces), and the air pollution has been frequently discussed as a sick house problem.
As a method of solving such sick house problem, there is given a method including suppressing generation sources of VOCs as contaminants and a method including removing generated VOCs with an air purifier.
As the former method, there is known a method called a bake-out method used for suppressing generation amounts of VOCs by keeping room temperature high, and recent scientific verification of the bake-out method have clarified the reality of its effects. However, the method requires the control of room temperature using a heating device, and hence the method is far from an available measure for residents to take.
Further, it is possible to use, for example, ethanol as a diluent or a solubilizer for VOCs, but the method is still unsatisfactory from the viewpoint of safety.
On the other hand, the latter method is used to remove VOCs at each time of their generation, but no measures are taken to cope with the generation sources of VOCs, and hence the method still includes many unsatisfactory points regarding the sick house problem.
Under such circumstances, there has already been provided, as the former method, for example, the technology described in Non Patent Literature 1.
This technology is used to suppress the generation of VOCs by spraying a special treatment agent (W'PHIXZ liquid) on the surfaces of building materials and furniture.
Further, there have already been provided, for example, the technologies described in Patent Literatures 1 and 2 in regard to the deodorization of air.
For example, Patent Literature 1 discloses, as a deodorant detergent product for kitchenware, one prepared by blending a surfactant, citric acid, malic acid, and eucalyptus essential oil with water. Further, Patent Literature 2 discloses, as a deodorant for purifying and deodorizing air, one prepared by adding cineol, which is a deodorant active ingredient obtained from plants, to a deodorant active ingredient extracted from forsythia and lactic acid.

### Citation List

#### Patent Literature

[PTL 1] JP 3597404 B2
[PTL 2] JP 2804823 B2

#### Non Patent Literature

[NPL 1] "Electricity and Construction" July 2003 issued by Ohmsha, Ltd., "Common knowledge about sick house problems that you have to know" on pages 101 to 106.

### Summary of Invention

#### Technical Problem

Admittedly, it is possible to suppress, to a certain extent, the generation of VOCs derived from building materials and furniture by using the technology described in Non Patent Literature 1, but the technology still involves an unsatisfactory point regarding deodorization.
From the standpoint of eliminating such inconvenience, it is possible to adopt, for example, a method in which the deodorant described in Patent Literature 1 or 2 is additionally used in combination with the treatment agent described in Non Patent Literature 1.
However, eachof the deodorants described in Patent Literatures 1 and 2 is used to provide a deodorant effect by adsorbing to obj ects, and hence its excessive scattering results in the remaining of the odor of the deodorant. Thus, those deodorants are capable of only changing the odors of VOCs generated from building materials and furniture to other smells rather than performing deodorization.
In addition, it is not clear whether each of the deodorants described in Patent Literatures 1 and 2 is compatible with the treatment agent described in Non Patent Literature 1, and some intrinsic functions of the treatment agent may be impaired by components of the deodorant.
A technical obj ect of the present invention is to provide a contaminant treatment agent that is capable of suppressing the emission of contaminants (such as volatile organic compounds contained in building materials and furniture) in living spaces into the outside and of providing a deodorant effect, a method of producing the contaminant treatment agent, and a method of using the contaminant treatment agent.

#### Means for solving the Problems

The invention according to claim 1 is a contaminant treatment agent for treating contaminants in living spaces, including: stable pure water which is excellent in oil component solubility and has a smaller time-dependent change in absorbance measured with a spectrophotometer under a condition of being left to stand for at least 5 hours or more than pure water comprising purified water, the stable pure water being prepared by irradiating pure water comprising neutral water free of occurrence of an electric charge imbalance with light collected by causing natural light to pass through a light collector using titanium or silicon; eucalyptus essential oil that is dissolved in the stable pure water and contains cineol as a main component; and a pre-selected mineral component that is added to the stable pure water.

The invention according to claim 2 is a contaminant treatment agent for treating contaminants in living spaces, including: stable pure water which is excellent in oil component solubility and has a characteristic of having a larger water contact angle on the same solid surface than pure water comprising purified water, the stable pure water being prepared by irradiating pure water comprising neutral water free of occurrence of an electric charge imbalance with light collected by causing natural light to pass through a light collector using titanium or silicon; eucalyptus essential oil that is dissolved in the stable pure water and contains cineol as a main component; and a pre-selected mineral component that is added to the stable pure water.

The invention according to claim 3 is a contaminant treatment agent for treating contaminants in living spaces according to claim 1, in which the stable pure water has a time-dependent change in absorbance of 0.02% or less measured with a spectrophotometer under the condition of being left to stand for at least 5 hours or more.

The invention according to claim 4 is a contaminant treatment agent for treating contaminants in living spaces according to any one of claims 1 to 3, in which a content of the eucalyptus essential oil is 0.02 to 0.1 part by weight with respect to 100 parts by weight of the stable pure water.

The invention according to claim 5 is a contaminant treatment agent for treating contaminants in living spaces according to any one of claims 1 to 4, in which the eucalyptus essential oil contains cineol at 90% or more.

The invention according to claim 6 is a method of producing a contaminant treatment agent for treating contaminants in living spaces as claimed in claim 1 or 2, the method including: a stable pure water production step of producing stable pure water; a stirring and mixing step of stirring and mixing eucalyptus essential oil containing cineol as a main component in the stable pure water produced in the stable pure water production step; and an addition step of adding a pre-selected mineral component to the stable pure water.

The invention according to claim 7 is a method of producing a contaminant treatment agent for treating contaminants in living spaces according to claim 6, in which the stable pure water production step includes: collecting natural light with a light collector formed of a sinter produced by sintering titanium particles or silicon particles; and irradiating pure water comprising neutral water free of occurrence of an electric charge imbalance with transmitted light passing through the light collector, thereby producing a base liquid which is the stable pure water.

The invention according to claim 8 is a method of using a contaminant treatment agent for treating contaminants in living spaces as claimed in claim 1 or 2, the method including spraying the contaminant treatment agent onto surfaces of building materials and furniture which are contaminant sources in living spaces.

The invention according to claim 9 is a method of using a contaminant treatment agent for treating contaminants in living spaces according to claim 8, the method further including spraying a pre-treatment agent comprising stable pure water and a pre-selected mineral component onto the surfaces of building materials and furniture which are contaminant sources in living spaces, prior to the spraying of the contaminant treatment agent.

The invention according to claim 10 is a liquid composition for constructing a contaminant treatment agent for treating contaminants in living spaces as claimed in claim 1 or 2, the liquid composition including the stable pure water and the eucalyptus essential oil.

Advantageous Effects of Invention

According to the invention according to claim 1 or 2, it is possible to suppress the emission of contaminants (such as volatile organic compounds contained in building materials and furniture) in living spaces into the outside and to provide a deodorant effect.

According to the invention according to claim 3, it is possible to suppress more certainly the emission of contaminants (such as volatile organic compounds contained in building materials and furniture) in living spaces into the outside, compared with the aspects not having the construction of the invention.

According to the invention according to claim 4, it is possible to more favorably maintain an adsorption retention effect on each of volatile organic compounds as contaminants and odor components, and solubility of eucalyptus essential oil with respect to stable pure water, compared with the aspects not having the construction of this invention.

According to the invention according to claim 5, it is possible to keep components more stable in an aqueous solution, to thereby more likely provide stability to its product, and moreover, to satisfactorily exert an adsorption retention effect on each of volatile organic compounds as contaminants and odor components, compared with the aspects not having the construction of this invention.

According to the invention according to claim 6, it is possible to produce a treatment agent capable of suppressing the emission of contaminants (such as volatile organic compounds contained in building materials and furniture) in living spaces into the outside and of providing a deodorant effect.

According to the invention according to claim 7, it is possible to produce a treatment agent capable of suppressing more certainly the emission of contaminants (such as volatile organic compounds contained in building materials and furniture) in living spaces into the outside, compared with the aspects not having the construction of this invention.

According to the invention according to claim 8, it is possible to spray the treatment agent according to the present invention efficiently on the surface to inside of each of building materials and furniture which are contaminant sources, and consequently, it is possible to suppress efficiently the emission of volatile organic compounds which are contaminants contained in the building materials and the like.

According to the invention according to claim 9, through the use of the pre-treatment agent including stable pure water and the pre-selected mineral component before the use of the contaminant treatment agent according to the present invention, it is possible to promote the emission and degradation of volatile organic compounds as contaminants that exhibit the emission tendency of volatilization (volatilization tendency) from the surfaces of building materials and furniture which are contaminant sources, thereby being able to clean the volatile organic compounds existing in the surfaces of the building materials and the like and being able to suppress efficiently the emission of volatile organic compounds that are present inside the building materials and the like.

According to the invention according to claim 10, the liquid composition can be used as a liquid composition for producing the contaminant treatment agent according to the present invention.

Brief Description of Drawings

[FIGS. 1(a), 1(b), and 1(c)] FIG. 1(a) is a schematic diagram illustrating an outline of an embodiment of a contaminant treatment agent (such as a VOC treatment agent) in a living space to which the present invention is applied, FIG. 1(b) is an explanatory diagram illustrating a method of producing the treatment agent, and FIG. 1(c) is an explanatory diagram illustrating a method of using the treatment agent.

[FIG. 2] FIG. 2 is an explanatory diagram illustrating an action of the VOC treatment agent serving as a contaminant treatment agent used in an embodiment.

[FIG. 3] FIG. 3 is an explanatory diagram illustrating a VOC treatment agent according to Embodiment 1.

[FIG. 4] FIG. 4 is a conceptual diagram schematically illustrating one example of a production apparatus for producing stable pure water which is a main component of the VOC treatment agent according to Embodiment 1.

[FIG. 5] FIG. 5 is an explanatory diagram illustrating one example of a method of producing the VOC treatment agent according to Embodiment 1.

[FIGS. 6(a) and 6(b)] FIG. 6(a) is an explanatory diagram illustrating one example of a method of using the VOC treatment agent used in Embodiment 1, and FIG. 6(b) is an explanatory diagram illustrating another method of using the VOC treatment agent.

[FIG. 7] FIG. 7 is an explanatory diagram illustrating one example of a performance evaluation apparatus for evaluating stable pure water which is a main component of a VOC treatment agent used in Example 1.

[FIGS. 8(a) and 8(b)] FIG. 8(a) is an explanatory diagram showing one example of performance evaluation results with respect to formaldehyde in Example 1, and FIG. 8(b) is an explanatory diagram showing one example of performance evaluation results with respect to other VOC components in Example 1.

[FIG. 9] FIG. 9 is an explanatory diagram illustrating a time-dependent change in absorbance characteristic of stable pure water measured with a spectrophotometer, the stable pure water being a main component of a VOC treatment agent used in Example 2.

[FIGS. 10(a) and 10(b)] FIGS. 10(a) and 10(b) are explanatory diagrams illustrating a time-dependent change in absorbance characteristic of ion-exchanged distilled water of Comparative Example 1 and that of tap water of Comparative Example 2, respectively, which are different from the stable pure water, the time-dependent changes being measured with a spectrophotometer.

[FIGS. 11(a) and 11(b)] FIG. 11(a) is an explanatory diagram showing surface tensions of stable pure water used in Example 3 and a sample of each of Comparative Examples 3, and FIG. 11(b) is an explanatory diagram showing water contact angles of Example 3 and each comparative example.

[FIG. 12] FIG. 12 is an explanatory diagram showing a dissolution characteristic of eucalyptus essential oil in stable pure water used in Example 4 and a dissolution characteristic of eucalyptus essential oil in water purified with a treatment agent used in Comparative Example 4, under the condition of containing eucalyptus essential oil at 0.05%.

[FIG. 13] FIG. 13 is an explanatory diagram showing a dissolution characteristic of eucalyptus essential oil in stable pure water used in Example 4 and a dissolution characteristic of eucalyptus essential oil in water purified with a treatment agent used in Comparative Example 4, under the condition of containing eucalyptus essential oil at 0.50%.

[FIG. 14] FIG. 14 is an explanatory diagram showing a dissolution characteristic of eucalyptus essential oil in stable pure water used in Example 4 and a dissolution characteristic of eucalyptus essential oil in water purified with a treatment agent used in Comparative Example 4, under the condition of containing eucalyptus essential oil at 1.00%.

[FIG. 15] FIG. 15 is an explanatory diagram illustrating one example of an experimental apparatus for measuring a time-dependent change in particle concentration in association with spraying of each of a VOC treatment agent used in Example 5 and water used in each of Comparative Examples 5.

[FIGS. 16(a) and 16(b)] FIG. 16(a) is an explanatory diagram showing the spray amount of each spray liquid, and FIG. 16(b) is an explanatory diagram illustrating a particle size distribution of the each spray liquid.

[FIG. 17] FIG. 17 is an explanatory diagram illustrating a time-dependent change in each particle concentration in a chamber in association with spraying of tap water.

[FIG. 18] FIG. 18 is an explanatory diagram illustrating a time-dependent change in each particle concentration in a chamber in association with spraying of purified water.

[FIG. 19] FIG. 19 is an explanatory diagram illustrating a time-dependent change in each particle concentration in a chamber in association with spraying of stable pure water A.

[FIG. 20] FIG. 20 is an explanatory diagram illustrating a time-dependent change in each particle concentration in a chamber in association with spraying of stable pure water B.

[FIG. 21] FIG. 21 is an explanatory diagram illustrating a time-dependent change in each particle concentration in a chamber in association with spraying of the VOC treatment agent according to Example 5.

[FIG. 22] FIG. 22 is an explanatory diagram illustrating a time-dependent change in each floating dust concentration in a chamber in association with spraying of an odoriferous substance treatment agent (deodorant) according to Example 6.

[FIG. 23] FIG. 23 is an explanatory diagram illustrating a time-dependent change in each floating dust concentration in a chamber in association with spraying of a deodorant according to Comparative Example 6-1.

[FIG. 24] FIG. 24 is an explanatory diagram illustrating a time-dependent change in each floating dust concentration in a chamber in association with spraying of a deodorant according to Comparative Example 6-2.

[FIG. 25] FIG. 25 is an explanatory diagram illustrating a time-dependent change in each floating dust concentration in a chamber in association with spraying of a deodorant according to Comparative Example 6-3.

[FIG. 26] FIG. 26 is an explanatory diagram illustrating a time-dependent change in each floating dust concentration in a chamber in association with spraying of a deodorant according to Comparative Example 6-4.

[FIG. 27] FIG. 27 is an explanatory diagram illustrating a time-dependent change in each floating dust concentration in a chamber in association with spraying of a deodorant according to Comparative Example 6-5.

[FIG. 28] FIG. 28 is an explanatory diagram illustrating a particle size distribution of each spray liquid used in Example 6 and each of Comparative Examples 6(6-1 to 6-5).

[FIG. 29] FIG. 29 is an explanatory diagram illustrating one example of an experimental apparatus for measuring a time-dependent change in ammonia concentration caused by an odoriferous substance treatment agent (deodorant) used in Example 7.

[FIG. 30] FIG. 30 is an explanatory diagram illustrating one example of a time-dependent change in ammonia concentration caused by an odoriferous substance treatment agent (deodorant) used in Example 7.

[FIG. 31] FIG. 31 is an explanatory diagram illustrating one example of a time-dependent change in ammonia concentration caused by an odoriferous substance treatment agent (deodorant) used in Comparative Example 7.

Description of Embodiments

Outline of embodiment

FIG. 1(a) illustrates an outline of an embodiment of a contaminant treatment agent such as a volatile organic compound treatment agent (hereinafter, abbreviated as VOC treatment agent if necessary) in a living space to which the present invention is applied.

Here, living spaces widely include room spaces for humans to live their lives, such as buildings, cars of trains and the like, large temporary tents, and underground malls.

In the figure, a VOC treatment agent S according to this embodiment is applied to the surfaces of building materials and furniture 2 in a living space such as a room space in a building 1, to thereby treat volatile organic compounds (VOCs) 3 contained in the building materials and furniture 2, and the VOC treatment agent S includes stable pure water 4 which is excellent in oil component solubility and has a smaller time-dependent change in absorbance measured with a spectrophotometer under the condition of being left to stand for at least 5 hours or more than pure water including purified water, the stable pure water 4 being prepared by irradiating pure water including neutral water free of the occurrence of an electric charge imbalance with light collected by causing natural light to pass through a light collector using titanium or silicon, eucalyptus essential oil 5 that is dissolved in the stable pure water 4 and contains cineol as a main component, and a pre-selected mineral component (not shown) that is added to the stable pure water 4.

Further, from another point of view, the VOC treatment agent S according to this embodiment is applied to the surfaces of the building materials and furniture 2 in a living space such as the room space in a building 1, to thereby treat the volatile organic compounds (VOCs) 3 contained in the building materials and furniture 2, and the VOC treatment agent S includes the stable pure water 4 which is excellent in oil component solubility and has the characteristic of having a larger water contact angle on the same solid surface than pure water including purified water, the stable pure water 4 being prepared by irradiating pure water including neutral water free of the occurrence of an electric charge imbalance with light collected by causing natural light to pass through a light collector using titanium or silicon, the eucalyptus essential oil 5 that is dissolved in the stable pure water 4 and contains cineol as a main component, and a pre-selected mineral component (not shown) that is added to the stable pure water 4.

In such technical means as described above, the term "stable pure water 4" means pure water which is stable as water. In general, the term "pure water" refers to water prepared by removing dissolved substances from natural water or tap water, such as ion-distilled purified water. However, when dissolved substances are removed from natural water or tap water, the resultant water has an active spot and is more liable to change as water, and hence the resultant water is more unstable than natural water or tap water containing dissolved substances.

Thus, the "stable pure water 4" is required to be more stable than the "pure water including purified water."

Here, a requirement for the stability of the "stable pure water 4" is, for example, to have a smaller time-dependent change in absorbance measured with a spectrophotometer than the "pure water including purified water, " or to have the characteristic of having a larger water contact angle on the same solid surface than the "pure water including purified water."

As described above, the potencies of the "stable pure water 4" are excellent impregnation property and excellent water retention property.

In addition, the "stable pure water 4" in this embodiment is required to be capable of dissolving a proper amount of the eucalyptus essential oil 5, and hence is required to be excellent in oil component solubility.

Further, it is good enough to use, as the eucalyptus essential oil 5, eucalyptus essential oil showing a dissolution characteristic with respect to the stable pure water 4 and containing cineol as a main component.

In addition, the potencies of cineol in the eucalyptus essential oil 5 include adsorbing to and retaining the VOC 3 or an odor component.

Here, the reasons for having selected the eucalyptus essential oil 5 are described.

In general, the following are given as problems of a deodorant based on an adsorption effect.

1) Odors are changed to other ones in the air rather than deodorization thereof.
2) When the deodorant is excessively scattered because its effect of deodorization and odor reduction is low, the odor of the deodorant may remain, leaving uncomfortable feeling.
3) A substance adsorbed and synthesized by the deodorant may remain and cause stickiness.
4) From the standpoint of the above-mentioned item 3), a coating may remain as a stain.
5) There are restrictions on the sites at which the deodorant is used and the objects to which the deodorant is applied.

In contrast, when the eucalyptus essential oil 5 is selected, the following advantages are offered.

1) It has strong diffusivity and permeability and is highly volatile.
2) It has an antifungal action and an antiviral action.
3) It has an antibacterial effect, and hence obviates the need for the use of artificial chemicals such as a preservative in a product.
4) It has a stable structure, includes cineol (such as 1, 8-cineol) at a large content, and easily dissolves in the stable pure water 4, and hence its stability after dissolution can be expected.
5) The eucalyptus essential oil 5 has good evaporation property with water after a product is scattered or coated and its odor does not remain, that is, no residual odor remains, no stickiness is caused, and stains are difficult to be produced.

Besides, it is good enough for the stable pure water 4, for example, to have a smaller time-dependent change in absorbance measured with a spectrophotometer than the "water including purified water, " and the stable pure water 4 preferably has a time-dependent change in absorbance of 0.02% or less measured with a spectrophotometer under the condition of being left to stand for at least 5 hours or more.

Further, the blend ratio of the eucalyptus essential oil 5, which may be selected appropriately, is preferably 0.02 to 0.1 part by weight with respect to 100 parts by weight of the stable pure water. Here, when the blend ratio of the eucalyptus essential oil 5 is less than 0.02 part by weight, its effect of adsorbing to and retaining the VOC 3 or an odor component may be weak, and when the blend ratio is more than 0.1 part by weight, its solubility with respect to the stable pure water 4 may be impaired. Inaddition, the eucalyptus essential oil 5 preferably contains cineol at 90% or more.

It is good enough for cineol to be contained as a main component, and a mode in which cineol is contained at 90% or more is preferred. This is because, when cineol is contained at a ratio of 90% or more, preferably more than 92%, the stability of the components is good in an aqueous solution, the stability of the resultant product is more likely to be retained, and the effect of adsorbing to and retaining the VOC 3 or an odor component is sufficiently exerted.

Further, when the above-mentioned VOC treatment agent S is produced, it is possible to use, as illustrated in FIG. 1(b), for example, a production method which includes a stable pure water production step of producing the stable pure water 4, a stirring and mixing step of stirring and mixing the eucalyptus essential oil 5 containing cineol as a main component in the stable pure water 4 produced in the stable pure water production step, and an addition step of adding a pre-selected mineral component (not shown) to the stable pure water 4.

Here, there is given, as a preferred mode of the stable pure water production step, a step including irradiating a light collector formedof a sinter produced by sintering titanium particles or silicon particles with natural light, and irradiating pure water including neutral water free of the occurrence of an electric charge imbalance with transmitted light passing through the light collector, thereby producing a base liquid as stable pure water.

Further, it is preferred to adopt, as a method of using the VOC treatment agent S, as illustrated in FIG. 1(c) for example, a method including spraying the VOC treatment agent S onto the surfaces of the building materials and furniture 2 in the room space in a building 1. This usage involves using, for example, an electric sprayer to subject the surfaces of objects to spray treatment.

Further, the VOC treatment agent S may be used alone in this embodiment, but the usage of the VOC treatment agent S is not limited thereto. It is possible to adopt, as illustrated by alternate long and two short dashed lines and solid lines in FIG. 1(c) for example, a method including spraying a VOC pre-treatment agent Sf including stable pure water and a pre-selected mineral component (not shown) onto the surfaces of the building materials and furniture 2 in the room space in a building 1, prior to spraying the VOC treatment agent S.

Next, it is described how the VOC treatment agent S used in this embodiment acts.

First, the mechanism of the production of the VOC 3 in the building materials and furniture 2 is described. As illustrated in FIG. 2, it has already been known that a main cause of the production of the VOC 3 is attributed to a plasticizer and a cross-linking agent contained in chemically synthesized new building materials or a preservative essential for wood building materials, those agents being inevitably used in modern architecture. Normal water (water containing a large content of natural water) used in each of those various kinds of chemical synthesis agents cannot serve as bonding water, which should be intrinsically stable in a building material 2a, and easily decays, leading to its evaporation. First, volatile hydrogen (H) having dissociated from$H_2O$ molecules start evaporating at a stretch while attracting various chemical substances. This "water decay" is the cause of the eternally continuing production of VOCs. On the other hand, oxidants M, which are compounds attributed to the dissociation of hydrogen (H) and show physical properties similar to those of ozone having a strong active force, induce an oxidization phenomenon in the building material 2a, producing substances that were not used at the time of the production of the building material 2a, and consequently, a degradation phenomenon may be caused in the building material 2a.

Those two actions are probably caused at the same time by the decay of "bonding water," and hence the VOC treatment agent S in this embodiment functions to stabilize the "bonding water, " thereby suppressing the production of the VOC 3 and the oxidization phenomenon in the building material 2a at the same time.

In this embodiment, in particular, the stable pure water 4 is water having excellent impregnation property and an excellent water retention ability, and besides, cineol in the eucalyptus essential oil 5 improves adsorption and retention property, thereby further promoting the stabilization of the "bonding water."

Note that in this embodiment, the contaminant treatment agent is not limited to the VOC treatment agent, and can be widely used as an odoriferous substance treatment agent for treating odoriferous substances (such as ammonia and methyl mercaptan) while keeping the same construction.

Embodiment 1

-VOC treatment agent-

FIG. 3 schematically illustrates a VOC treatment agent, which is a contaminant treatment agent, according to Embodiment 1.

In the figure, the VOC treatment agent S is prepared by, for example, stirring and mixing, in a container 10, stable pure water 11 being more stable than pure water including purified water and eucalyptus essential oil 12 containing cineol as a main component.

In this embodiment, there is used, as the stable pure water 11, stable pure water exhibiting a time-dependent change in absorbance of 0.02% or less measured with a spectrophotometer under the condition of being left to stand for at least 5 hours or more in an absorbance measurement with the spectrophotometer.

Here, the absorbance measurement test with a spectrophotometer used in this embodiment is described.

In this test, absorbance is measured with a spectrophotometer and a time-dependent change in absorbance is observed.

1) Unused cells are used to confirm a margin of error. The same kind of pure water is measured in each cell to confirm a margin of error among the cells.
2) A specimen is measured in the each cell in the above-mentioned item 1).
3) The each cell containing the specimen measured in the above-mentioned item 2) is taken out from the spectrophotometer and is stored as it is.

In order to store the each cell containing the specimen, the cell is put on a flat place and is shielded with, for example, a glass beaker which has been washed with pure water and dried, thereby reducing the influence of an indoor air flow or the like. 4) A time-dependent change is measured.

The each cell containing the specimen is left to stand for a predetermined time period (for example, 5 hours or more) and is subjected to measurement again as it is.

In this case, a specimen having a time-dependent change in absorbance of 0.02% or less measured with a spectrophotometer is estimated to be stable pure water.

Note that in another method of confirming a specimen to be the stable pure water 11, the water contact angle of a specimen is measured and when the specimen at least has a larger water contact angle compared with that of pure water including purified water, the specimen is estimated to be the stable pure water 11.

Further, in this embodiment, there is used, as the eucalyptus essential oil 12, eucalyptus essential oil containing cineol 12a at more than 92%, and the eucalyptus essential oil is blended in the stable pure water 11 at a blend ratio of 0.02 to 0.1 part by weight with respect to 100 parts by weight of the stable pure water.

-Stable pure water production step-

FIG. 4 is a conceptual diagram schematically illustrating one example of a production apparatus 20 for producing the stable pure water 11.

In the figure, 22 denotes a hemispherical light collector made of titanium. The light collector 22 is formed of a sinter produced by sintering titanium particles. In this case, it is suitable to use a sinter produced by sintering a compact in which titanium particles each having a larger particle diameter (such as a particle diameter of 50 μm) are arranged in the outermost side of the hemispherical portion, titanium particles each having a medium particle diameter (such as a particle diameter of 20 μm) are arranged inside the outermost side, and titanium particles each having a smaller particle diameter (such as a particle diameter of 10 μm) are arranged in the innermost side. Note that a sinter produced by sintering silicon particles may be used for the light collector 22, but because silicon with high purity is expensive, it is more advantageous to use a sinter produced by sintering titanium particles.

Then, below the light collector 22 (its spherical surface side facing downward), a glass tank 26 formed of high-purity $SiO_2$ is arranged. In the glass tank 26, pure water 28 prepared by treatment with an ion-exchange resin is fed.

Here, natural water with good quality is selected for the pure water 28 so as to prepare neutral water free of the occurrence of an electric charge imbalance in water. In this case, it is important to carefully select water (water source) having the perfect balance (quality and amounts of cations and anions). If water having a large electric charge imbalance is used, pure water prepared by water-purification treatment is inferior in stability as water. Further, because the pure water 28 is stored in the high-purity glass tank 26, the motion of water (oscillatory and rotary movements of a water molecule) on the basis of an electrostatic interaction is more stabilized.

In the ground below the glass tank 26, provided are a layer 30 formed of silicon particles each having a high purity (such as 96 to 98%) and having a relatively large particle diameter (the layer including, for example, particles with a diameter of 5 to 6 mm and having a thickness of 1,000 mm) in the outermost layer side, a layer 32 formed of silicon particles each having a medium particle diameter (the layer including, for example, particles with a diameter of 2 to 3 mm and having a thickness of 500 mm) beneath the outermost layer, and a layer 34 formed of silicon particles each having a smaller particle diameter (the layer including, for example, particles with a diameter of 1 mm or less and having a thickness of 300 mm) as the bottom layer.

The light collector 22 and the glass tank 26 are each supported with a suitable supporting member (not shown). The height of the light collector 22 from the surface of the ground varies depending on the treatment amount of the pure water 28 and is set to about 3 to 6 m. The glass tank 26 is disposed in the middle of the height.

After settings are made as described above, the light collector 22 is irradiated with natural light to collect efficiently natural light energy (light corpuscles with 200 nm or less are effective in this case). Titanium or silicon allows light having a certain wavelength component in natural light to transmit. The transmitted light passes through the pure water 28 in the glass tank 26, and the transmitted light removes water molecules that tend to evaporate and decay easily, thereby purifying the pure water 28 into more stable pure water (stable pure water) 11.

In this case, the ground layer formed of high-purity silicon particles is provided in the surface of the ground and the glass tank 26 is provided above the ground layer, and hence it is particularly possible to increase the transmission efficiency of light energy.

After that, the energy collection stable time of the light corpuscles is confirmed and treated water is taken out from the glass tank 26. Unless the treated water is taken out at the collection stable time (time at which stable molecules having the same volume among water molecules gather), the stability of the treated water cannot be expected afterward.

The stability of the treated water is confirmed and the treated water whose stability has been confirmed is stored as the stable pure water 11 in an original water storage (not shown).

In order to confirm the stability, the above-mentioned absorbance measurement with a spectrophotometer is typically used, but it is possible to use any of other methods (such as electric conductivity measurement, oxidation-reduction potential measurement, and electron diffraction measurement).

Further, it is recommended that trace amounts of elements (for example, mineral components such as selenium, cesium, potassium, and krypton) be blended depending on the purposes (depending on the reactions of substance components), thereby completing a product using the stable pure water 11.
The following four formulations are exemplified.

1) The bond between hydrogen and a hydroxyl group in substance components is reinforced.
2) Substance components are fragmented (into single molecules) to perform stabilization.
3) The strength of each hydrogen bond in raw material water (use water) is reinforced to suppress the oxidization phenomenon attributed to the dissociation of hydrogen bonds. The decay of water due to an environmental change is prevented. Note that even if any of other physical actions (ultraviolet rays, heating, cooling, electromagnetic waves, and the like) is applied to perfect stable molecule-gathering water, the effects of the water do not change.
4) Substance components are firmly covered with hydrocolloids or protective colloids to prevent hazardous substance actions. Here, a perfect stable molecule-gathering body covers all substances including hydrophobic substances, providing hydrophilicity to all the substances. As a result, a water vessel phenomenon is caused by water molecules excellent in a gathering force.

-Method of producing VOC treatment agent-

Next, FIG. 5 illustrates one example of a method of producing a VOC treatment agent.
The method of producing a VOC treatment agent is described with reference to the figure. After a dedicated tank line is subjected to usual cleaning, the tank is filled with a predetermined amount (such as 800 liters) of pure water, and stable pure water in which a mineral component is blended is then added. After that, the whole is left to stand at normal temperature for a predetermined time period (for example, a stable time is set to 1 hour), and eucalyptus essential oil is then added, for example, at 0.05%. Then, the resultant is stirred with a dedicated stirring member provided in the tank for a predetermined time period (such as 10 minutes), a bacterial test is carried out after a predetermined waiting time period (such as 48 hours) elapses, and the resultant VOC treatment agent is filled in a container. Note that, when the VOC treatment agent is filled in a container, a predetermined amount (such as about 50 liters) of the VOC treatment agent is run for line cleaning, and then the filling work of the VOC treatment agent is carried out.

-Use method-

FIG. 6(a) illustrates a method of using the VOC treatment agent of this embodiment.
According to the figure, the VOC treatment agent S is sprayed with an electric sprayer 60 on the surfaces of building materials and furniture 15 in a room, thereby being able to decompose VOCs such as formaldehyde arising from the building materials and furniture 15, resulting in the reduction of the amounts of the VOCs.
In this case, it is preferred that spray conditions C for the electric sprayer 60 be set so that the VOC treatment with the spray liquid can be attained most efficiently, by suitably selecting a spray speed v and a spray amount Q. Taking the spray amount Q as an example, a spray amount of 10 to 25 ml/$m^2$ is suitable based on the spraying of a liquid containing fine particles with a diameter of 5 to 10 $\mu$m.
Here, the building materials include fittings such as doors and walls, plywood, laminated wood, rugs, and carpets, and the treatment agent may be sprayed preliminarily on those products in the factories thereof. When the treatment agent is sprayed on furniture, it is preferred to adjust the degree of atomization of water in a base liquid which is stable pure water. This is because, if there is used a base liquid including water with a large degree of atomization and having a too large momentum, the action of the base liquid is so large that the painted surfaces of painted furniture are roughened, and moreover, as various kinds of chemicals such as a wood preservative, an insect repellent, an anti-fungal agent, and a plasticizer are used for furniture, the base liquid eventually reduces the functions of these chemicals. It is recommended that the degree of atomization a base liquid be adjusted depending on the types of those chemicals. The degree of atomization is adjusted by adjusting the treatment time of the stabilization treatment described in FIG. 4. On the other hand, the VOC treatment agent S is considered to have, in contrast, the function of suppressing the generation of volatile components, when the VOC treatment agent S penetrates into the building materials and furniture 15. Chemicals used for the building materials and furniture 15 include many chlorine-based or bromine-based substances binding to benzene rings, and the VOC treatment agent S is capable of strengthening the binding force of these binding substances, thereby probably being able to suppress the volatilization of hazardous components.
Besides, the VOC treatment agent S in this embodiment contains mineral components (such as selenium, cesium, potassium, and krypton) in a base liquid which is stable pure water, and hence it is expected that the mineral components strongly act on VOCs, decomposing the VOCs.
Moreover, the VOS treatment agent S in this embodiment includes eucalyptus essential oil containing cineol as a main component, and hence cineol adsorbs to and retains VOCs inside the building materials and furniture 15. In addition, because eucalyptus essential oil easily evaporates together with water, the odor components of the eucalyptus essential oil do not remain in the building materials and furniture 15 for an unnecessarily long period.
Besides, in this embodiment, it is a matter of course that the VOS treatment agent S can be used alone, but as illustrated in FIG. 6(b), it is also possible to use the VOC pre-treatment agent Sf including a base liquid which is stable pure water and a pre-selected mineral component before using the VOS treatment agent S. Here, examples of the VOC pre-treatment agent Sf include the following.

1) Decomposition liquid: a base liquid (at 99% or more) and selenium, potassium, palladium, and krypton (each in an amount of less than 0.001 mg/L)
2) Chemical treatment liquid: a base liquid (at 99% or more) and cesium, potassium, and tantalum (each in an amount of less than 0.001 mg/L)
3) Finishing liquid: a base liquid (at 99% or more) and selenium, potassium, and palladium (each in an amount of less than 0. 001 mg/L)
4) Stabilizing liquid 1: a base liquid (at 99% or more) and cesium, potassium, hafnium, and krypton (each in an amount of less than 0.001 mg/L)
5) Stabilizing liquid 2: a base liquid (at 99% or more) and bromine, boron, chromium, and potassium (each in an amount of less than 0. 001 mg/L)

Here, the decomposition liquid is used for the purpose of decomposing and removing VOCs produced in the surfaces of building materials and furniture, the chemical treatment liquid is used for the purpose of treating chemicals harmlessly, the finishing liquid is used for the purpose of finishing the surfaces of buildingmaterials and furniture, and the stabilizing liquids 1 and 2 are used for the purpose of stably retaining VOCs inside building materials and furniture and are separately used depending on the materials of building materials (a painted surface, a cloth surface, and the like).

Examples

Example 1

Taking the VOC treatment agent used in Embodiment 1, which is a contaminant treatment agent, as Example 1, FIG. 7 illustrates one example of a performance evaluation apparatus for evaluating stable pure water which is a main component of Example 1.

In the figure, a performance evaluation apparatus 100 includes a small chamber 101 in accordance with ISO classification and is structured so that, after the inside of the small chamber 101 is cleaned and ventilated, a sample (specimen) 102 treated with stable pure water which is a main component of the VOC treatment agent is placed in the small chamber 101, a scavenging tube 105 formed of a DNPH cartridge is used to scavenge formaldehyde among VOCs, a carbon-based scavenging tube 106 is used to scavenge other VOCs, and the scavenged formaldehyde and other components are analyzed with flow meters 107 and 108 and gas meters 109 and 110, respectively. Note that in FIG. 7, reference numeral 111 denotes a cleaning tube and reference numerals 112 and 113 denote pumps.

The results of the analysis are determined by placing a specimen in the small chamber, scavenging each chemical substance after one day, three days, and seven days have passed, measuring the concentration of the scavenged chemical substance, and calculating a chemical substance emission rate EF and the reduction ratio of the emission rate, to thereby evaluate the performance of stable pure water which is a main component of the used VOC treatment agent.

Here, the chemical substance emission rate EF is calculated by substituting the measured concent rations (C, $C_0$) into the following equation (1):

$$EF=Q(C-C_0)/A \qquad (1)$$

where EF represents an emission rate (ωg/$m^2$· h), C represents a concentration in a chamber (ωg/$m^3$), $C_0$ represents a concentration in supplied air (ωg/$m^3$), Q represents a ventilation amount ($m^2$/h), and A represents a surface area of a sample ($m^3$).

Further, it is possible to determine the reduction ratio of the emission rate by, for example, comparing emission rates before and after spraying.

FIGS. 8(a) and 8(b) show the performance evaluation results of stable pure water which is a main component of the VOC treatment agent according to this example.

The results in the figures were obtained on the basis of the condition that a VOC pre-treatment agent (which was constructed of a decomposition liquid, a finishing liquid, and a stabilizing liquid 1 in this case) was used as a product using stable pure water, and test pieces of a floor, a wall, a ceiling, and furniture were prepared as specimens by using their members, provided that "countermeasure building materials" prepared by applying spray treatment to some of these test pieces with the VOC pre-treatment agent were used as specimens of this example and, on the other hand, "blank building materials" that were some of these test pieces to which spray treatment had not been applied were used as comparative specimens.

Further, FIG. 8(a) shows formaldehyde emission rates with respect to each specimen and FIG. 8(b) shows emission rates of other VOCs with respect to, for example, a floor as a specimen. "Mist" refers to a specimen to which spray treatment was applied and "Blank" refers to a specimen to which spray treatment was not applied.

The results in the figures have confirmed the effect of reducing a formaldehyde emission rate and the effect of reducing emission rates of other VOCs in the case of using stable pure water which is a main component of the VOC treatment agent according to this example, and have confirmed that spraying stable pure water which is a main component of the VOC treatment agent can suppress a VOC emission rate.

Example 2

Taking the VOC treatment agent according to Embodiment 1 as Example 2, FIG. 9 illustrates a time-dependent change in absorbance characteristic of stable pure water used in Example 2 measured with a spectrophotometer. Note that the time-dependent change in absorbance herein is measured in accordance with the test method described in Embodiment 1.

According to the figure, it is understood that the time-dependent change in absorbance of the stable pure water is kept within 0.02%.

In contrast, it is understood that the time-dependent changes in absorbance of Comparative Example 1 (distilled water) and Comparative Example 2 (tap water) are large, as illustrated in FIGS. 10(a) and 10(b), compared with that of the stable pure water.

Example 3

Taking the VOC treatment agent according to Embodiment 1 as Example 3, FIGS. 11(a) and 11(b) show the results obtained by examining the surface tensions and water contact angles (such as a water contact angle on a chromium-plated plate) of stable pure waters A and B used in Example 3 and each comparative example (pure water, tap water, and xylene).

Note that the stable pure water A corresponds to stable pure water used in a decomposition liquid serving as the VOC pre-treatment agent Sf, and the stable pure water B corresponds to stable pure water used in a stabilizing liquid serving as the VOC pre-treatment agent Sf.

According to the figures, it is understood that the water contact angles of the stable pure waters A and B are larger than those of pure water and tap water, and it is understood that the surface tensions of the stable pure waters A and B are smaller than that of pure water.

Example 4

Taking the VOC treatment agent according to Embodiment 1 as Example 4, FIGS. 12 to 14 show the results obtained by examining the solubility of eucalyptus essential oil in stable pure water in Example 4. Note that the solubility of eucalyptus essential oil in purified water was examined as Comparative Example 4 in order to compare with Example 4.

FIGS. 12 to 14 show the results obtained by setting the blend ratio of eucalyptus essential oil to 0.05%, 0.50%, and 1.00%, respectively. In FIG. 12, it was confirmed that eucalyptus essential oil in Example 4 completely dissolved after 72 hours, and the solution exhibited transparency, whereas eucalyptus essential oil in Comparative Example 4 was further separated from water after 72 hours, resulting in the occurrence of a floating oil film. Further, in FIG. 13 and FIG. 14, it was conf irmed that eucalyptus essential oil in Example 4 completely dissolved after 72 hours, exhibiting increased transparency, whereas eucalyptus essential oil in Comparative Example 4 was further separated from water after 72 hours, resulting in the occurrence of a floating oil film or the increase of an oil film.

As a result, it was confirmed that the solubility of eucalyptus essential oil in stable pure water in Example 4 was very good when the blend ratio of eucalyptus essential oil was 0.05% to 1.00%.

Example 5

Taking the VOC treatment agent according to Embodiment 1 as Example 5 (eucalyptus essential oil containing cineol at 95% is used in this case), FIG. 15 illustrates an experimental apparatus for performing spraying tests of the VOC treatment agent serving as Example 5 and each of Comparative Examples 5 (5-1 to 5-4).

An experimental apparatus 120 in the figure is structured so that the treatment liquidof eachof Example 5 and Comparative Examples 5 is sprayed with a sprayer 122 in a large chamber 121 in accordance with ISO classification, and each particle concentration in association with the spraying of the treatment liquid in the large chamber 121 is measured with a particle counter (such as a light scattering automatic particle counter) 123.

FIG. 16(a) shows the spray amounts of the treatment liquids of Example 5 and each of Comparative Examples 5, and FIG. 16(b) illustrates a particle size distribution of each of the spray liquids.

FIG. 17 to FIG. 21 each illustrate a time-dependent change in each particle concentration in the chamber in association with the spraying of each treatment liquid of Example 5 and each of Comparative Examples 5 (5-1 to 5-4).

According to FIG. 17 to FIG. 21, the maximum rise in the particle concentration immediately after the spraying of the VOC treatment agent in Example 5 is smaller compared with that in each of Comparative Examples 5.

As illustrated in FIG. 16, this VOC treatment agent is **characterized in that**, when it is sprayed horizontally toward a wall surface, the increase tendency of, in particular, the concentration of floating particulate substances having a particle diameter of 0.3 μm (floating dust concentration) is obviously smaller compared with those of tap water and purified water.

That is, clean air was supplied into the large chamber, the inside of the chamber was controlled to certain environmental conditions (ventilation rate: 0.01 (1/h), temperature: 23°C, relative humidity: 50%, and airflow rate: less than 0.01 (m/s)), and each of spraying agents of tap water, purified water, stable pure water A, stable pure water B, and Example 5 was sprayed in a given amount by using an electric sprayer toward a position at a distance of about 2 meters in the chamber wall surface direction from the outside of the chamber. As a result, the increase tendency of the concentration of floating particulate substances per 1 g of spray amount is clearly different among the spraying agents.

In particular, the difference among the spraying agents is large in regard to particles having a particle diameter

of 0.3 μm. Note that the concentrations per unit spray amount (particles/L·g) of tap water, purified water, stable pure water A, stable pure water B, and Example 5 are 620, 291, 379, 330, and 288.

Example 6

In this example, taking an odoriferous substance treatment agent (deodorant) having the same construction as the VOC treatment agent according to Embodiment 1 as Example 6 (eucalyptus essential oil containing cineol at 95% is used in this case), the same experimental apparatus 120 as in Example 5 was used to carry out spray tests.

FIG. 22 to FIG. 27 each illustrate a time-dependent change in a particle concentration in the chamber in association with the spraying of each treatment liquid of Example 6 and each of Comparative Examples 6 (6-1 to 6-5). Further, FIG. 28 illustrates particle size distributions of Example 6 and each of Comparative Examples 6. Note that the concentration per unit spray amount (particles/L·g) in FIG. 28 is expressed in a logarithmic scale.

As illustrated in FIG. 23 to FIG. 27, when the agent of each of Comparative Examples 6 (each is a commercially available deodorant) is sprayed from its respective container (sprayer) in the chamber, the increase tendency of the concentrations of floating particulate substances having different particle diameters (floating dust concentrations) varies depending on the deodorants (Comparative Examples 6). In contrast, as illustrated in FIG. 22, the increase of the concentrations of particles having diameters of 0.3 μm, 0.5 μm, 1. μm, 2.0 μm, and 5.0 μm hardly occurs in the case of Example 6.

That is, clean air was supplied into the large chamber, the inside of the chamber was controlled to certain environmental conditions (ventilation rate: 0.01 (1/h), temperature: 23°C, relative humidity: 50%, and airflow rate: less than 0.01 (m/s)), and each deodorant was sprayed in a given amount by using a sprayer toward the center of the chamber from the outside of the chamber. As a result, no increase was observed in concentration in the case of Example 6, but the increase tendency of the concentration of floating particulate substances per 1 g of spray amount was clearly different among the deodorants according to Comparative Examples 6.

As described above, it is understood according to this example that the particle concentration in the chamber hardly changes between before and after the spraying of the deodorant. This means that even if the deodorant is sprayed, the number of floating particles hardly increases.

In contrast, each of Comparative Examples 6 (6-1 to 6-5) shows such a time-dependent change that the number of floating particles sharply increases immediately after the spraying of the deodorant and then gradually decreases with time, which fact is largely different from this example.

Example 7

Taking an odoriferous substance treatment agent (deodorant) having the same construction as the VOC treatment agent according to Embodiment 1 as Example 7, FIG. 29 illustrates an experimental apparatus for examining a time-dependent change in ammonia concentration in association with the use of the VOC treatment agent of Example 7 (eucalyptus essential oil containing cineol at 95% is used in this case).

An experimental apparatus 130 in the figure is structured so that, after the inside of a large chamber 131 in accordance with ISO classification is cleaned with an air cleaning unit 132, ammonia gas in a gas cylinder 133 is taken into the large chamber 131 in a predetermined amount by using an integrating flowmeter 134, followed by stirring with a stirring fan 135, a deodorant is sprayed from a sprayer 136, and the ammonia gas is trapped in an impinger 137, followed by measurement with an integrating flowmeter 138. Note that reference numeral 139 denotes a constant flow pump.

FIG. 30 is a graph chart illustrating a time-dependent change in ammonia concentration in association with the spraying of the deodorant of Example 7.

FIG. 31 is a graph chart illustrating a time-dependent change in ammonia concentration in association with the spraying of the deodorant of Comparative Example 7 (a commercially available deodorant is used).

Note that conditions for each of Example 7 and Comparative Example 7 are such that the volume of the large chamber is 4.98 ($m^3$), the temperature in the large chamber is 28±1 (°C), the relative humidity is 50±1 (%), the airflow rate is 0.2 to 0.3 (m/s), and the ventilation rate is 0.01 (1/h).

According to those figures, the ammonia concentration shows a time-dependent decrease after the spraying of each deodorant.

Particularly in Example 7, as illustrated in FIG. 30, the ammonia concentration in the chamber clearly decreases immediately after the spraying (the ammonia concentration ($mg/m^3$) is 2.08 at the start of the spraying and decreases to 1.83 after 5 minutes), and then the concentration reduces at a larger decrease ratio than the decrease ratio before the spraying (from "-0.0034" to "-0.0039"). Note that when the ammonia removal ratio of the deodorant in this case is represented by an equivalent ventilation volume $Q_{eq}$ of an index obtained by multiplying an equivalent ventilation rate by a chamber air volume, the ammonium equivalent ventilation volume comes to 0.09 ($m^3/h$). On the other hand, in Comparative Example 7, as illustrated in FIG. 31, the ammonia concentration in the chamber clearly decreases immediately after the spraying, but the concentration only reduces afterward at a smaller decrease ratio than the decrease ratio before the spraying (from "-0.0058" to "-0.0015"). In this case, the ammonium equivalent ventilation volume is almost 0 ($m^3/h$), and hence it has been confirmed by the calculations that there is a large difference in ammonium removal performance between the both.

Here, the equivalent ventilation volume is a volume obtained by converting the target contaminant removal performance of an air cleaner or a deodorant to a room ventilation volume. For example, when a deodorant has an ammonium equivalent ventilation volume of 1 ($m^3$/h), the fact means that the deodorant has the performance to be able to supply 1 ($m^3$/h) of fresh air containing no ammonia into a room in one hour (see "Latest knowledge for preventing sick house problems, Architectural Institute of Japan, Gihodo Shuppan Co., Ltd., pp. 196-208, March 2005").

Reference Signs List

1 ··· room space in building, 2 ··· building material and furniture, 3 ··· VOC, 4 ··· stable pure water, 5 ··· eucalyptus essential oil, S ··· VOC treatment agent, Sf ··· VOC pre-treatment agent

## Claims

**1.** A contaminant treatment agent for treating contaminants in living spaces, comprising:

stable pure water which is excellent in oil component solubility and has a smaller time-dependent change in absorbance measured with a spectrophotometer under a condition of being left to stand for at least 5 hours or more than pure water comprising purified water, the stable pure water being prepared by irradiating pure water comprising neutral water free of occurrence of an electric charge imbalance with light collected by causing natural light to pass through a light collector using titanium or silicon;
eucalyptus essential oil that is dissolved in the stable pure water and contains cineol as a main component; and
a pre-selected mineral component that is added to the stable pure water.

**2.** A contaminant treatment agent for treating contaminants in living spaces, comprising:

stable pure water which is excellent in oil component solubility and has a characteristic of having a larger water contact angle on the same solid surface than pure water comprising purified water, the stable pure water being prepared by irradiating pure water comprising neutral water free of occurrence of an electric charge imbalance with light collected by causing natural light to pass through a light collector using titanium or silicon;
eucalyptus essential oil that is dissolved in the stable pure water and contains cineol as a main component; and
a pre-selected mineral component that is added to the stable pure water.

**3.** A contaminant treatment agent for treating contaminants in living spaces according to claim 1, wherein the stable pure water has a time-dependent change in absorbance of 0.02% or less measured with a spectrophotometer under the condition of being left to stand for at least 5 hours or more.

**4.** A contaminant treatment agent for treating contaminants in living spaces according to any one of claims 1 to 3, wherein a content of the eucalyptus essential oil is 0.02 to 0.1 part by weight with respect to 100 parts by weight of the stable pure water.

**5.** A contaminant treatment agent for treating contaminants in living spaces according to any one of claims 1 to 4, wherein the eucalyptus essential oil contains cineol at 90% or more.

**6.** A method of producing a contaminant treatment agent for treating contaminants in living spaces as claimed in claim 1 or 2, the method comprising:

a stable pure water production step of producing stable pure water;
a stirring and mixing step of stirring and mixing eucalyptus essential oil containing cineol as a main component in the stable pure water produced in the stable pure water production step; and
an addition step of adding a pre-selected mineral component to the stable pure water.

**7.** A method of producing a contaminant treatment agent for treating contaminants in living spaces according to claim 6,
wherein the stable pure water production step comprises:

collecting natural light with a light collector formed of a sinter produced by sintering titanium particles or silicon particles; and
irradiating pure water comprising neutral water free of occurrence of an electric charge imbalance with transmitted light passing through the light collector, thereby producing a base liquid which is the stable pure water.

**8.** A method of using a contaminant treatment agent for treating contaminants in living spaces as claimed in claim 1 or 2, the method comprising spraying the contaminant treatment agent onto surfaces of building materials and furniture which are contaminant sources in living spaces.

**9.** A method of using a contaminant treatment agent for treating contaminants in living spaces according to claim 8, the method further comprising spraying a pre-treatment agent comprising stable pure water and a pre-selected mineral component onto the surfaces of building materials and furniture which are contaminant sources in living spaces, prior to the spraying of the contaminant treatment agent.

**10.** A liquid composition for constructing a contaminant treatment agent for treating contaminants in living spaces as claimed in claim 1 or 2, the liquid composition comprising the stable pure water and the eucalyptus essential oil.

FIG.1

(a)

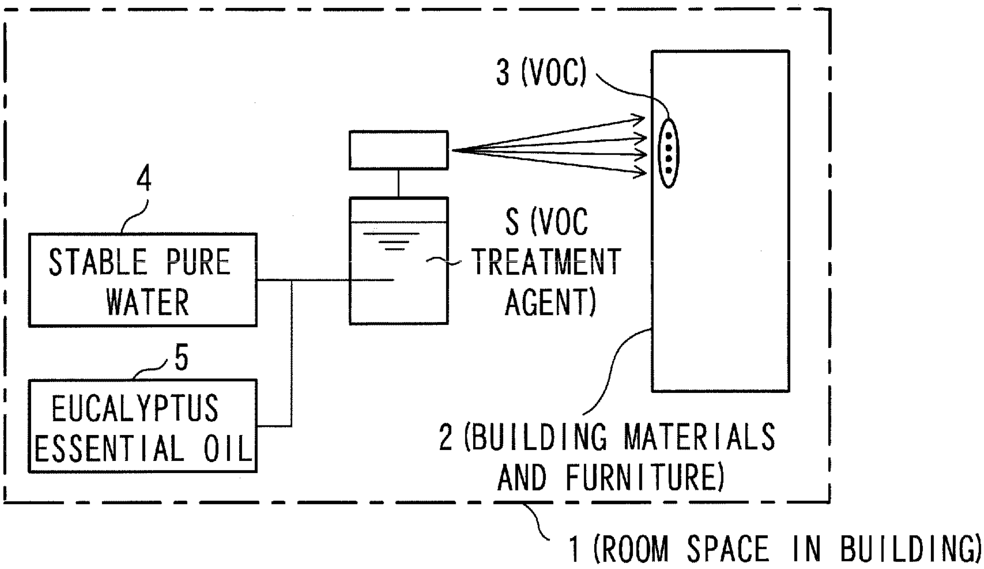
3 (VOC)
4
STABLE PURE WATER
S (VOC TREATMENT AGENT)
5
EUCALYPTUS ESSENTIAL OIL
2 (BUILDING MATERIALS AND FURNITURE)
1 (ROOM SPACE IN BUILDING)

(b)

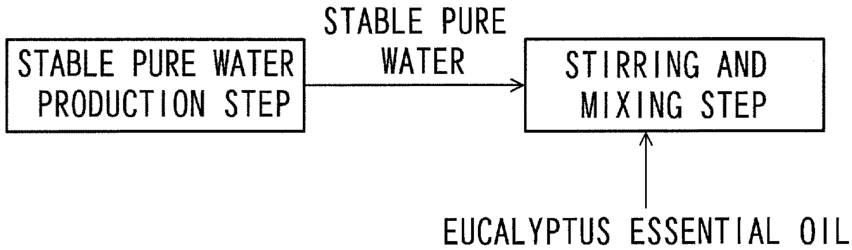
STABLE PURE WATER PRODUCTION STEP
STABLE PURE WATER
STIRRING AND MIXING STEP
EUCALYPTUS ESSENTIAL OIL

(c)

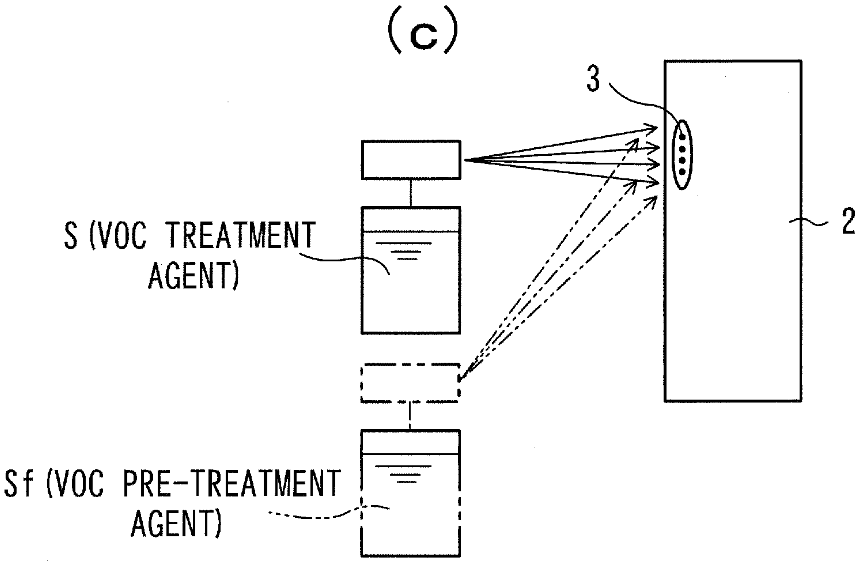
3
2
S (VOC TREATMENT AGENT)
Sf (VOC PRE-TREATMENT AGENT)

FIG.2

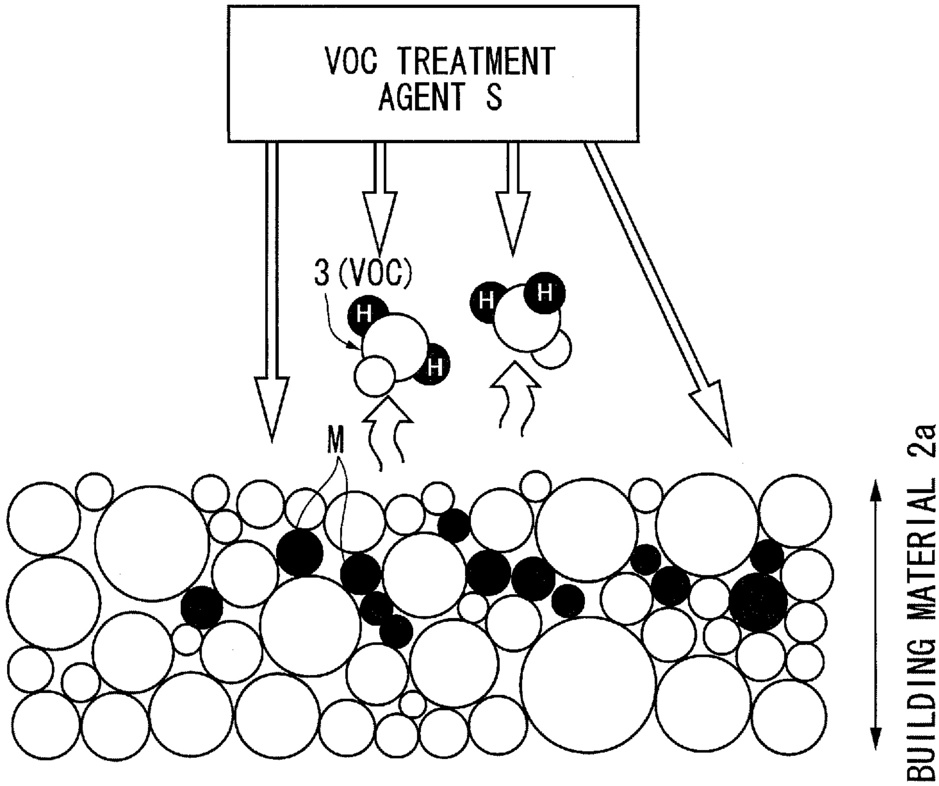

FIG.3

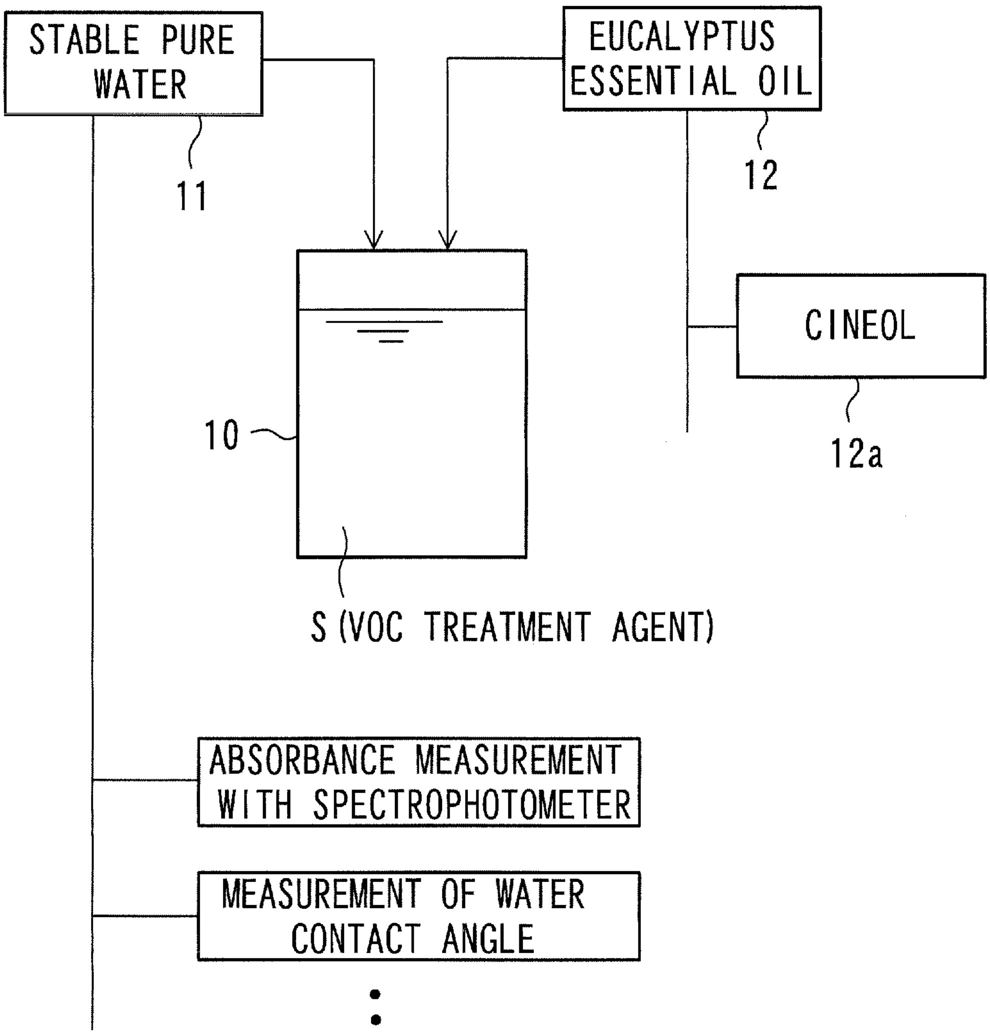
STABLE PURE WATER
11
EUCALYPTUS ESSENTIAL OIL
12
CINEOL
12a
10
S (VOC TREATMENT AGENT)
ABSORBANCE MEASUREMENT WITH SPECTROPHOTOMETER
MEASUREMENT OF WATER CONTACT ANGLE

FIG.4

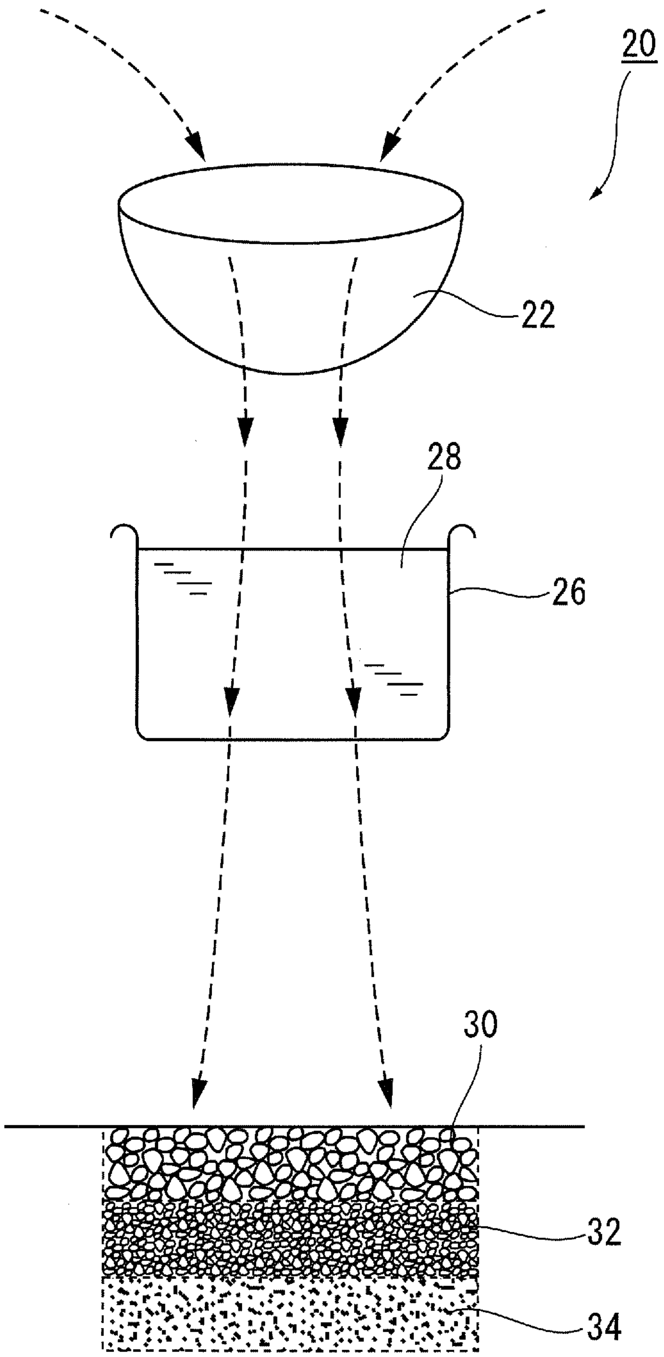

FIG.5

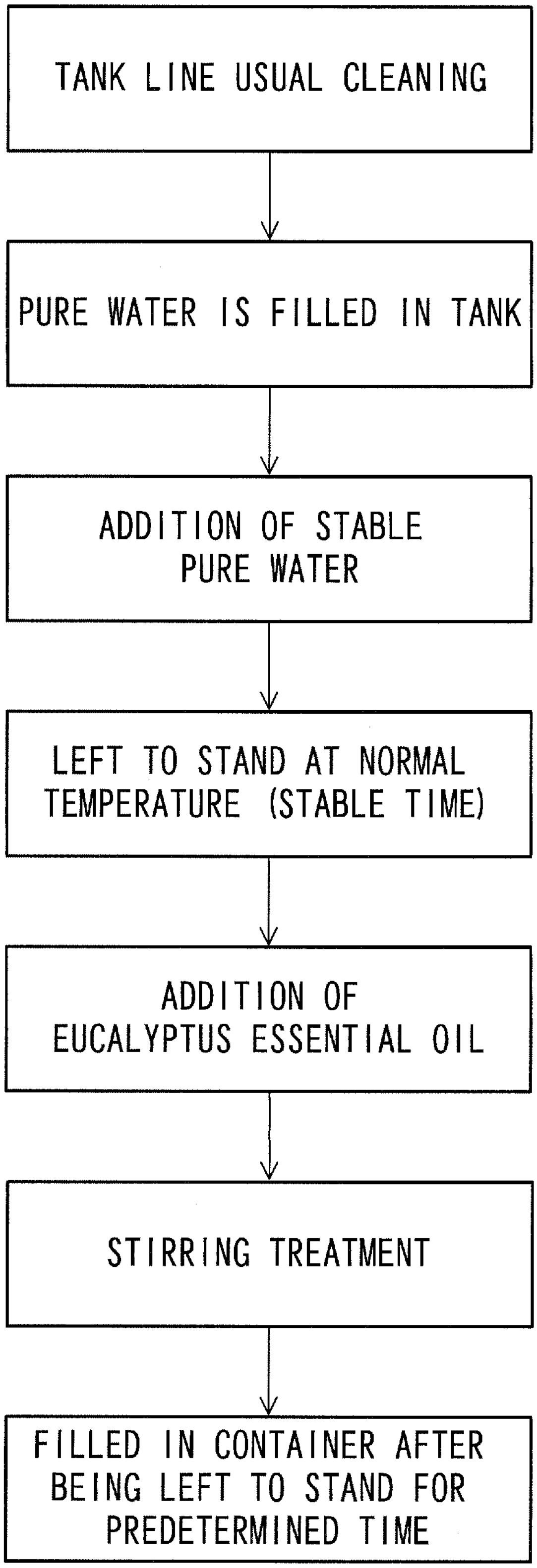
TANK LINE USUAL CLEANING
PURE WATER IS FILLED IN TANK
ADDITION OF STABLE PURE WATER
LEFT TO STAND AT NORMAL TEMPERATURE (STABLE TIME)
ADDITION OF EUCALYPTUS ESSENTIAL OIL
STIRRING TREATMENT
FILLED IN CONTAINER AFTER BEING LEFT TO STAND FOR PREDETERMINED TIME

FIG.6

(a)

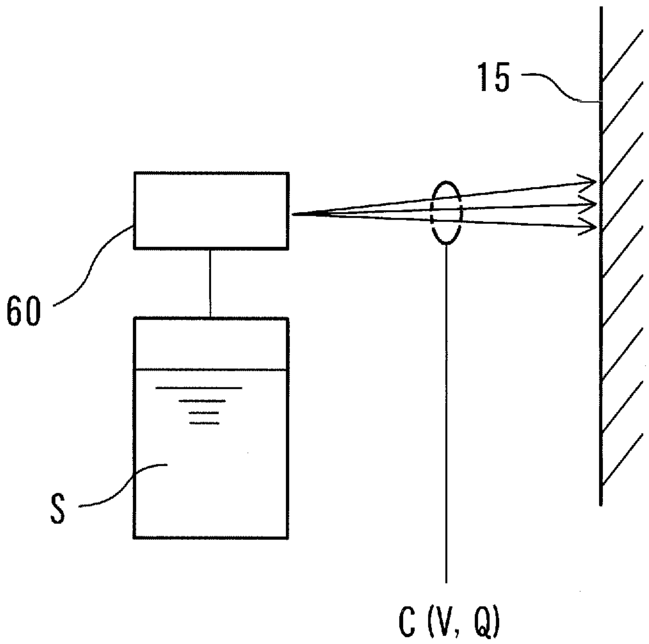
15
60
S
C (V, Q)

(b)

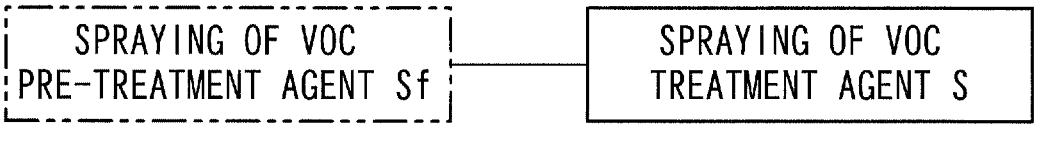
SPRAYING OF VOC
PRE-TREATMENT AGENT Sf
SPRAYING OF VOC
TREATMENT AGENT S

FIG.7

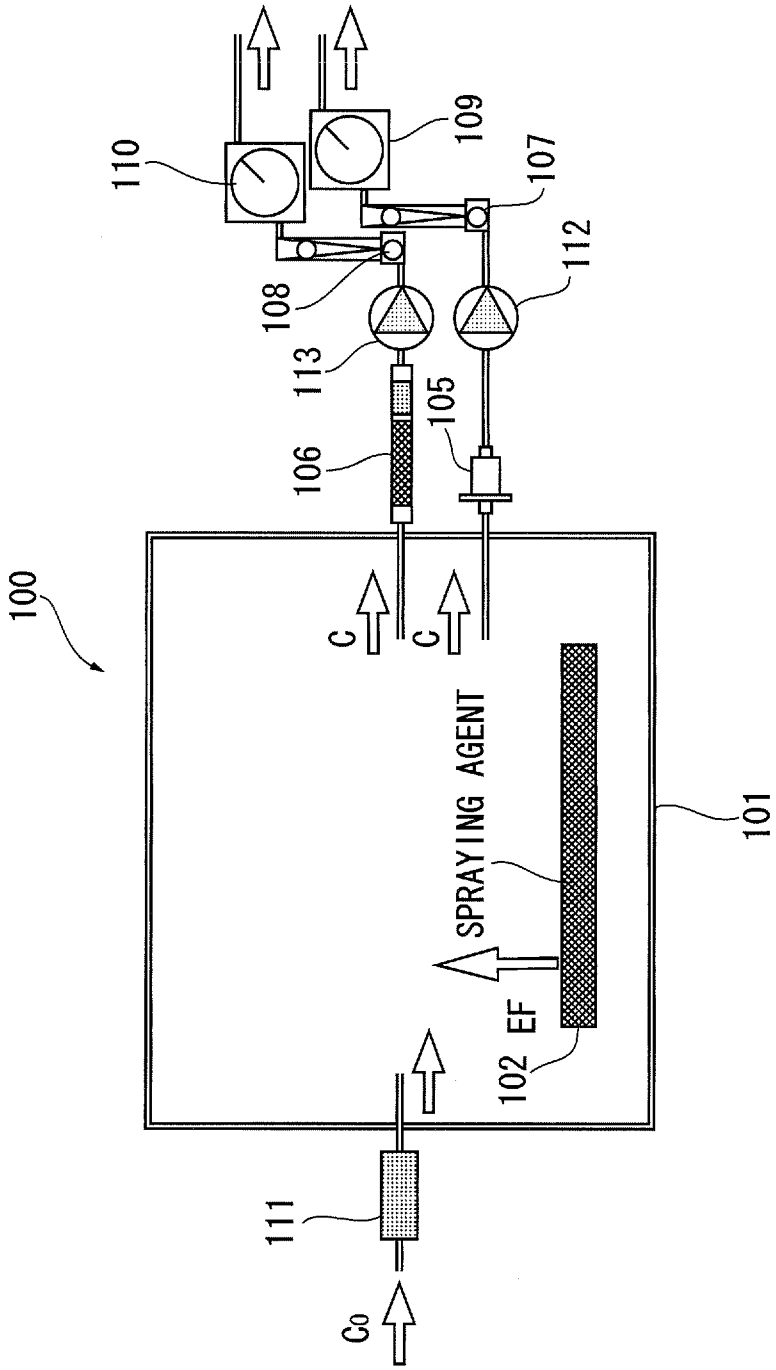
100
110
109
107
112
108
113
105
106
C
C
SPRAYING AGENT
101
EF
102
111
$C_0$

FIG.8

(a) FORMALDEHYDE EMISSION RATE

| SPECIMEN | | EMISSION RATE EF[μ g/m²·h] | | | |
|---|---|---|---|---|---|
| | | BEFORE COATING | AFTER COATING | | |
| | | | ONE DAY LATER | THREE DAYS LATER | SEVEN DAYS LATER |
| FLOOR | Blank | 2.37 | 1.29 | 2.42 | 1.76 |
| | Mist | 2.67 | 0.722 | 1.26 | 0.880 |
| WALL | Blank | 2.88 | 4.28 | 4.13 | 2.91 |
| | Mist | 2.63 | 1.93 | 2.25 | 1.11 |
| CEILING | Blank | 17.5 | 11.3 | 10.5 | 9.05 |
| | Mist | 16.2 | 9.39 | 7.77 | 6.57 |
| FURNITURE | Blank | 9.30 | 7.59 | 6.8 | 6.77 |
| | Mist | 9.45 | 5.21 | 4.76 | 4.54 |

(b) VOC EMISSION RATE (FLOOR)

| VOC COMPONENT | | EMISSION RATE EF[μ g/m²·h] | | | |
|---|---|---|---|---|---|
| | | BEFORE COATING | AFTER COATING | | |
| | | | ONE DAY LATER | THREE DAYS LATER | SEVEN DAYS LATER |
| Ethanol | Blank | 995 | 927 | 606 | 486 |
| | Mist | 871 | 388 | 289 | 191 |
| Acetone | Blank | 297 | 262 | 243 | 206 |
| | Mist | 244 | 231 | 177 | 133 |
| 2-propanol | Blank | 29.9 | 23.8 | 20.0 | 20.6 |
| | Mist | 26.4 | 16.8 | 13.8 | 10.6 |
| Methylethylketone | Blank | 14.8 | 11.3 | 12.2 | 10.3 |
| | Mist | 11.4 | 10.3 | 8.99 | 8.86 |
| Hexane | Blank | 47.9 | 38.2 | 31.4 | 25.5 |
| | Mist | 59.5 | 27.9 | 20.1 | 13.06 |
| Benzene | Blank | 9.99 | 9.42 | 9.32 | 8.05 |
| | Mist | 9.83 | 6.64 | 5.09 | 3.83 |
| Toluene | Blank | 55.3 | 52.6 | 58.7 | 43.4 |
| | Mist | 52.9 | 33.8 | 39.5 | 34.6 |
| Octane | Blank | 256 | 233 | 214 | 207 |
| | Mist | 233 | 170 | 125 | 112 |
| Ethylbenzene | Blank | 17.6 | 22.2 | 19.0 | 22.7 |
| | Mist | 22.6 | 17.3 | 8.37 | 11.3 |
| m,p-xylene | Blank | 73.6 | 59.3 | 65.7 | 54.5 |
| | Mist | 63.8 | 32.8 | 30.6 | 31.9 |
| Styrene | Blank | 15.4 | 12.8 | 13.2 | 10.7 |
| | Mist | 12.3 | 5.27 | 5.34 | 5.32 |
| o-xylene | Blank | 32.8 | 28.0 | 39.6 | 32.4 |
| | Mist | 32.1 | 17.1 | 19.1 | 21.1 |
| m,p-ethyltoluene | Blank | 44.7 | 38.5 | 42.1 | 42.9 |
| | Mist | 45.0 | 18.5 | 25.4 | 21.5 |
| 1,2,4-trimethylbenzene | Blank | 53.4 | 35.7 | 23.2 | 21.9 |
| | Mist | 46.0 | 25.6 | 17.2 | 16.0 |
| Nonanal | Blank | 14.3 | 15.0 | 17.4 | 12.5 |
| | Mist | 14.6 | 13.1 | 13.6 | 5.39 |

# FIG.9

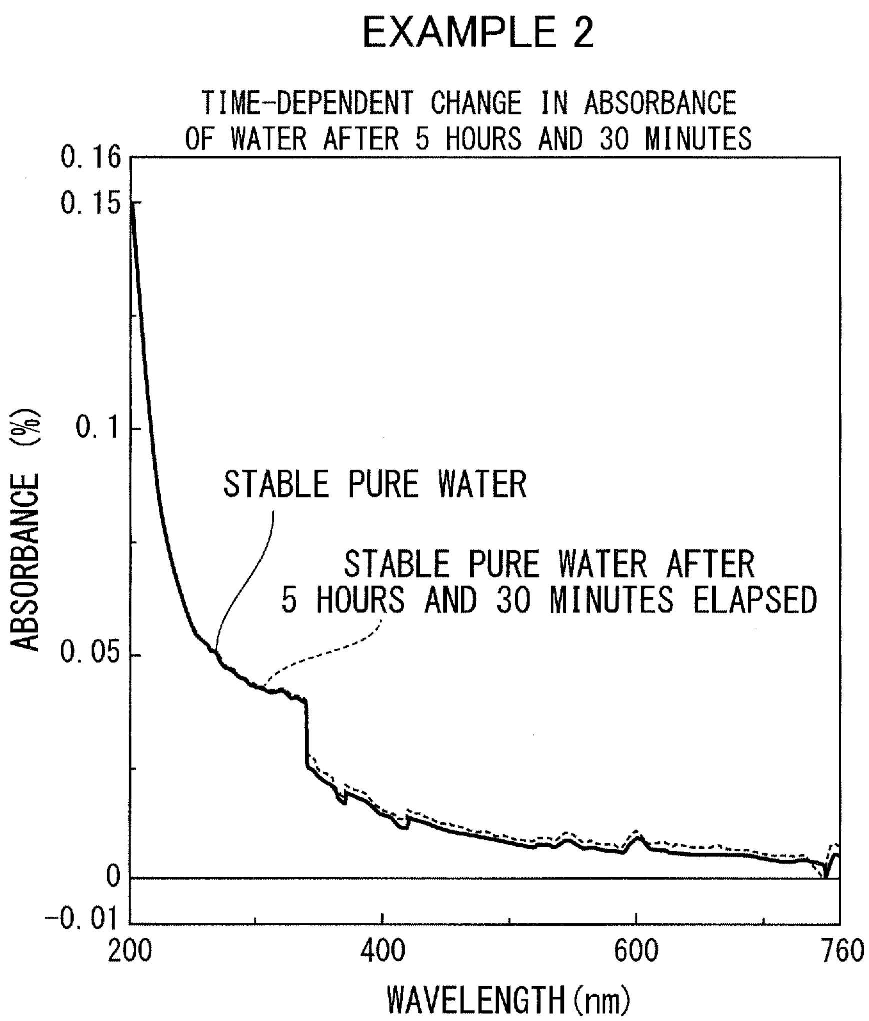
EXAMPLE 2
TIME-DEPENDENT CHANGE IN ABSORBANCE
OF WATER AFTER 5 HOURS AND 30 MINUTES
0.16
0.15
0.1
0.05
0
-0.01
ABSORBANCE (%)
STABLE PURE WATER
STABLE PURE WATER AFTER
5 HOURS AND 30 MINUTES ELAPSED
200
400
600
760
WAVELENGTH (nm)

FIG.10

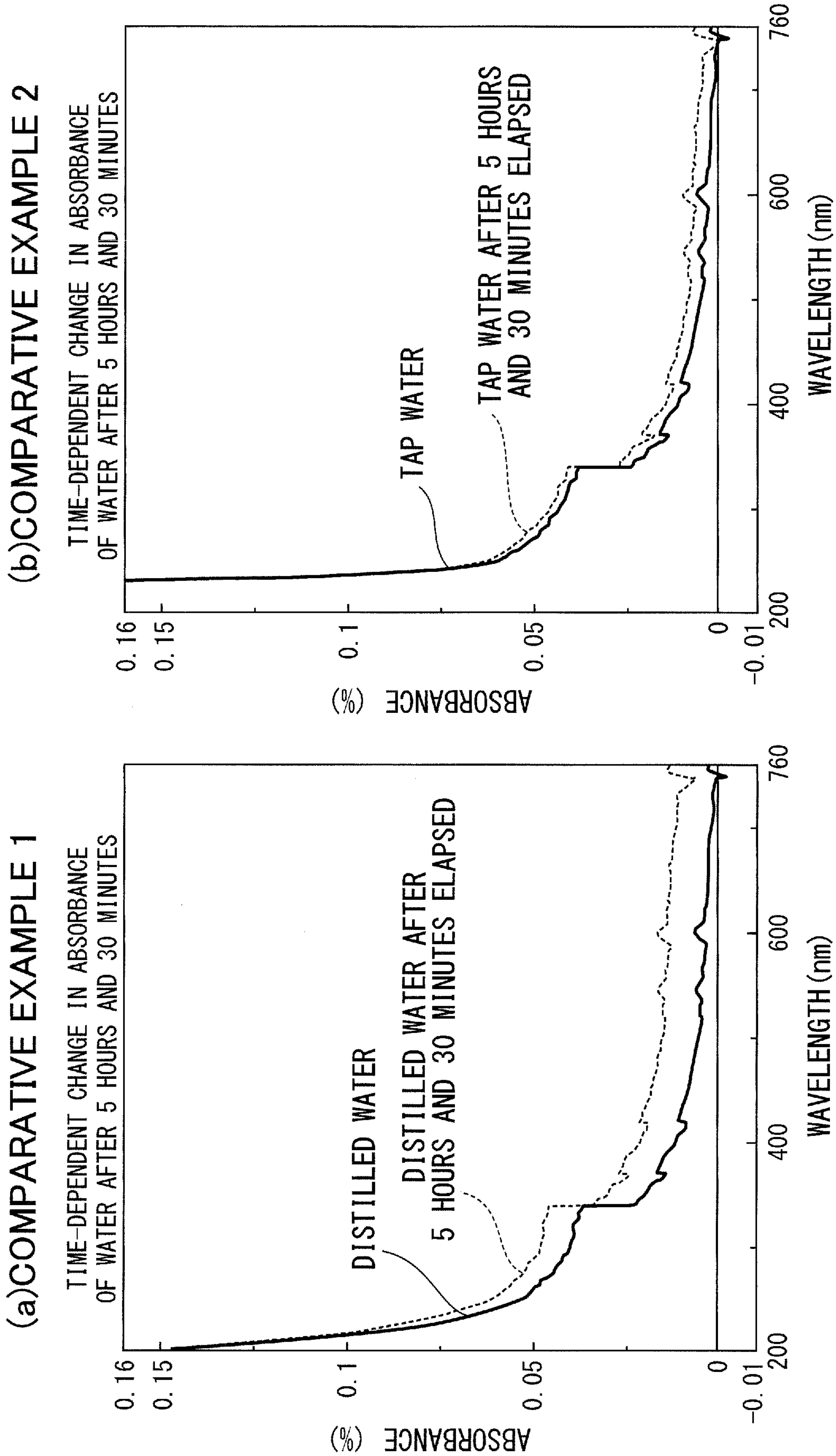
(a)COMPARATIVE EXAMPLE 1
TIME-DEPENDENT CHANGE IN ABSORBANCE OF WATER AFTER 5 HOURS AND 30 MINUTES
DISTILLED WATER
DISTILLED WATER AFTER 5 HOURS AND 30 MINUTES ELAPSED
ABSORBANCE (%)
0.16
0.15
0.1
0.05
0
-0.01
200
400
600
760
WAVELENGTH (nm)
(b)COMPARATIVE EXAMPLE 2
TIME-DEPENDENT CHANGE IN ABSORBANCE OF WATER AFTER 5 HOURS AND 30 MINUTES
TAP WATER
TAP WATER AFTER 5 HOURS AND 30 MINUTES ELAPSED
ABSORBANCE (%)
0.16
0.15
0.1
0.05
0
-0.01
200
400
600
760
WAVELENGTH (nm)

FIG.11

(a) SURFACE TENSION

| SAMPLE | SURFACE TENSION |
|---|---|
| ①PURE WATER | 54. 2dyne/cm |
| ②TAP WATER | 45. 4dyne/cm |
| ③STABLE PURE WATER A | 51. 0dyne/cm |
| ④STABLE PURE WATER B | 50. 0dyne/cm |
| ⑤XYLENE | 28. 7dyne/cm |

(b) WATER CONTACT ANGLE

| SAMPLE | CHROMIUM-PLATED PLATE |
|---|---|
| ①PURE WATER | 86° |
| ②TAP WATER | 85° |
| ③STABLE PURE WATER A | 89° |
| ④STABLE PURE WATER B | 90° |

# FIG.12

## EUCALYPTUS ESSENTIAL OIL 0.05%

| ELAPSE OF TIME | COMPARATIVE EXAMPLE 4 (PURIFIED WATER) | EXAMPLE 4 (STABLE PURE WATER) | EVALUATION RESULTS |
|---|---|---|---|
| EARLY TIME | 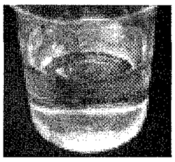 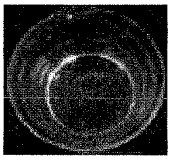 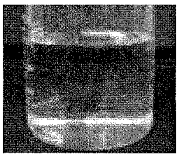 | 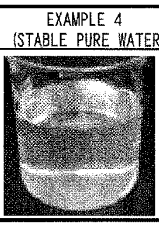 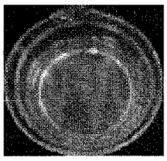 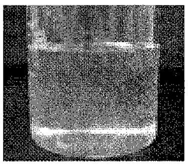 | PURIFIED WATER: AN OIL FILM IS FLOATING.<br>STABLE PURE WATER:<br>EUCALYPTUS ESSENTIAL OIL COMPLETELY DISSOLVES. |
| SECOND PHOTOGRAPHING AFTER 24 HOURS | 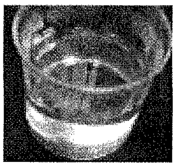 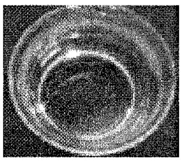 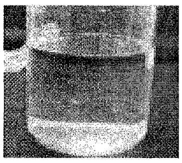 | 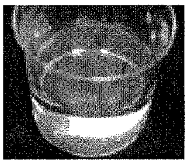 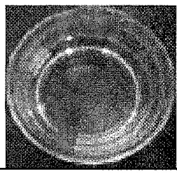 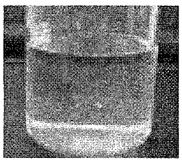 | PURIFIED WATER:<br>EUCALYPTUS ESSENTIAL OIL IS SEPARATED FROM WATER AND AN OIL FILM IS FLOATING.<br>STABLE PURE WATER:<br>THE EUCALYPTUS ESSENTIAL OIL COMPLETELY DISSOLVES AND THE SOLUTION IS MORE TRANSPARENT. |
| THIRD PHOTOGRAPHING AFTER 72 HOURS | 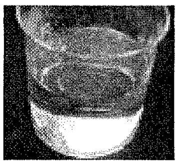 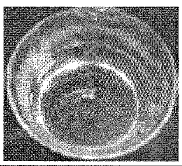 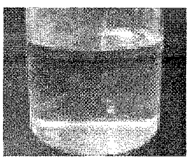 | 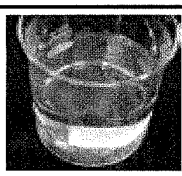 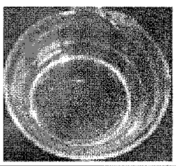 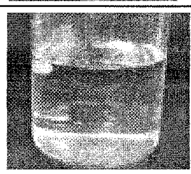 | PURIFIED WATER:<br>THE EUCALYPTUS ESSENTIAL OIL IS FURTHER SEPARATED FROM WATER AND AN OIL FILM IS FLOATING.<br>STABLE PURE WATER:<br>THE EUCALYPTUS ESSENTIAL OIL COMPLETELY DISSOLVES AND THE SOLUTION IS TRANSPARENT. |

# FIG.13

## EUCALYPTUS ESSENTIAL OIL 0.50%

| ELAPSE OF TIME | COMPARATIVE EXAMPLE 4 (PURIFIED WATER) | EXAMPLE 4 (STABLE PURE WATER) | EVALUATION RESULTS |
|---|---|---|---|
| | 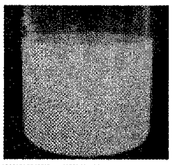 | 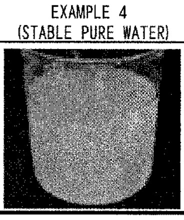 | |
| EARLY TIME | 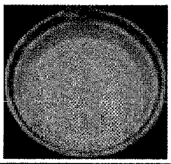 | 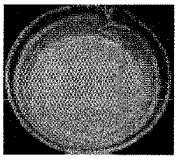 | |
| | 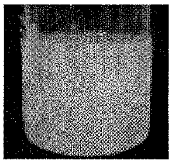 | 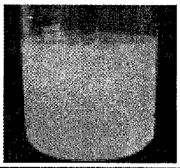 | PURIFIED WATER: THE SOLUTION IS CLOUDY AND AN OIL FILM IS FLOATING. STABLE PURE WATER: THE SOLUTION IS CLOUDY AND EUCALYPTUS ESSENTIAL OIL COMPLETELY DISSOLVES. |
| | 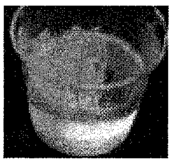 | 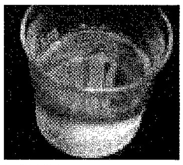 | |
| SECOND PHOTOGRAPHING AFTER 24 HOURS | 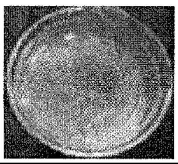 | 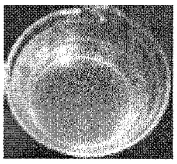 | |
| | 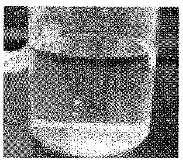 | 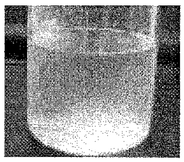 | PURIFIED WATER: EUCALYPTUS ESSENTIAL OIL IS SEPARATED FROM WATER AND AN OIL FILM IS FLOATING. STABLE PURE WATER: THE EUCALYPTUS ESSENTIAL OIL COMPLETELY DISSOLVES AND THE SOLUTION IS MORE TRANSPARENT. |
| | 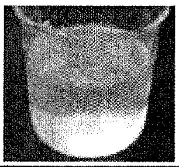 | 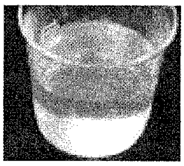 | |
| THIRD PHOTOGRAPHING AFTER 72 HOURS | 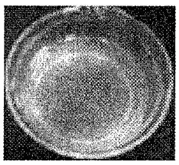 | 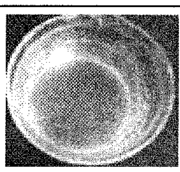 | |
| | 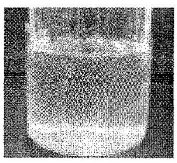 | 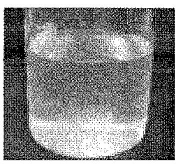 | PURIFIED WATER: THE EUCALYPTUS ESSENTIAL OIL IS FURTHER SEPARATED FROM WATER AND AN OIL FILM IS FLOATING. STABLE PURE WATER: THE EUCALYPTUS ESSENTIAL OIL COMPLETELY DISSOLVES STABLY AND THE SOLUTION IS MORE TRANSPARENT. |

# FIG.14

## EUCALYPTUS ESSENTIAL OIL 1.00%

| ELAPSE OF TIME | COMPARATIVE EXAMPLE 4 (PURIFIED WATER) | EXAMPLE 4 (STABLE PURE WATER) | EVALUATION RESULTS |
|---|---|---|---|
| EARLY TIME | 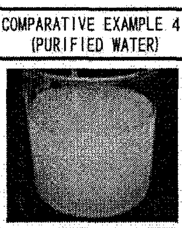 | 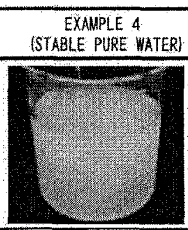 | |
| | 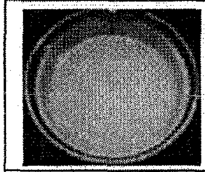 | 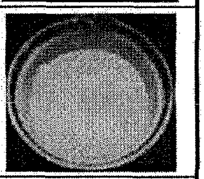 | |
| | 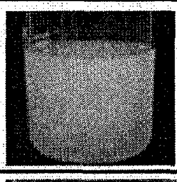 | 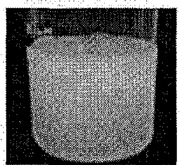 | PURIFIED WATER: THE SOLUTION IS CLOUDY AND AN OIL FILM IS FLOATING. STABLE PURE WATER: THE SOLUTION IS CLOUDY AND A SMALL AMOUNT OF OIL FILM IS FLOATING. |
| SECOND PHOTOGRAPHING AFTER 24 HOURS | 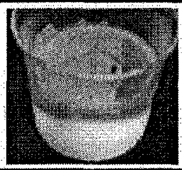 | 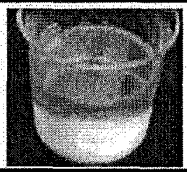 | |
| | 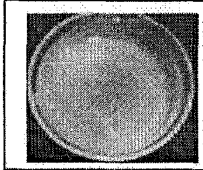 | 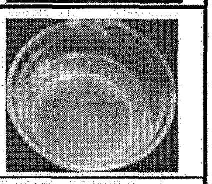 | |
| | 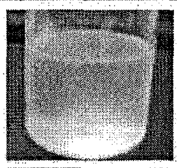 | 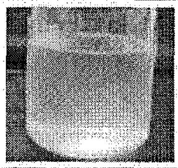 | PURIFIED WATER: EUCALYPTUS ESSENTIAL OIL IS SEPARATED FROM WATER AND THE AMOUNT OF THE OIL FILM IS LARGER. STABLE PURE WATER: EUCALYPTUS ESSENTIAL OIL DISSOLVES AND THE SOLUTION IS MORE TRANSPARENT. |
| THIRD PHOTOGRAPHING AFTER 72 HOURS | 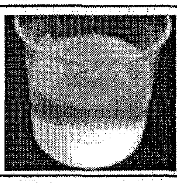 | 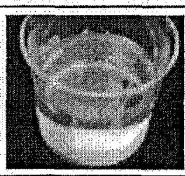 | |
| | 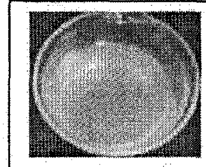 | 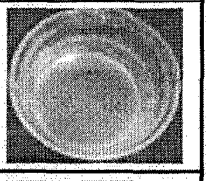 | |
| | 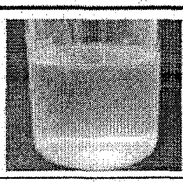 | 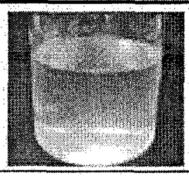 | PURIFIED WATER: THE EUCALYPTUS ESSENTIAL OIL IS FURTHER SEPARATED FROM WATER AND THE AMOUNT OF THE OIL FILM IS LARGER. STABLE PURE WATER: THE EUCALYPTUS ESSENTIAL OIL COMPLETELY DISSOLVES AND THE SOLUTION IS MORE TRANSPARENT. |

FIG.15

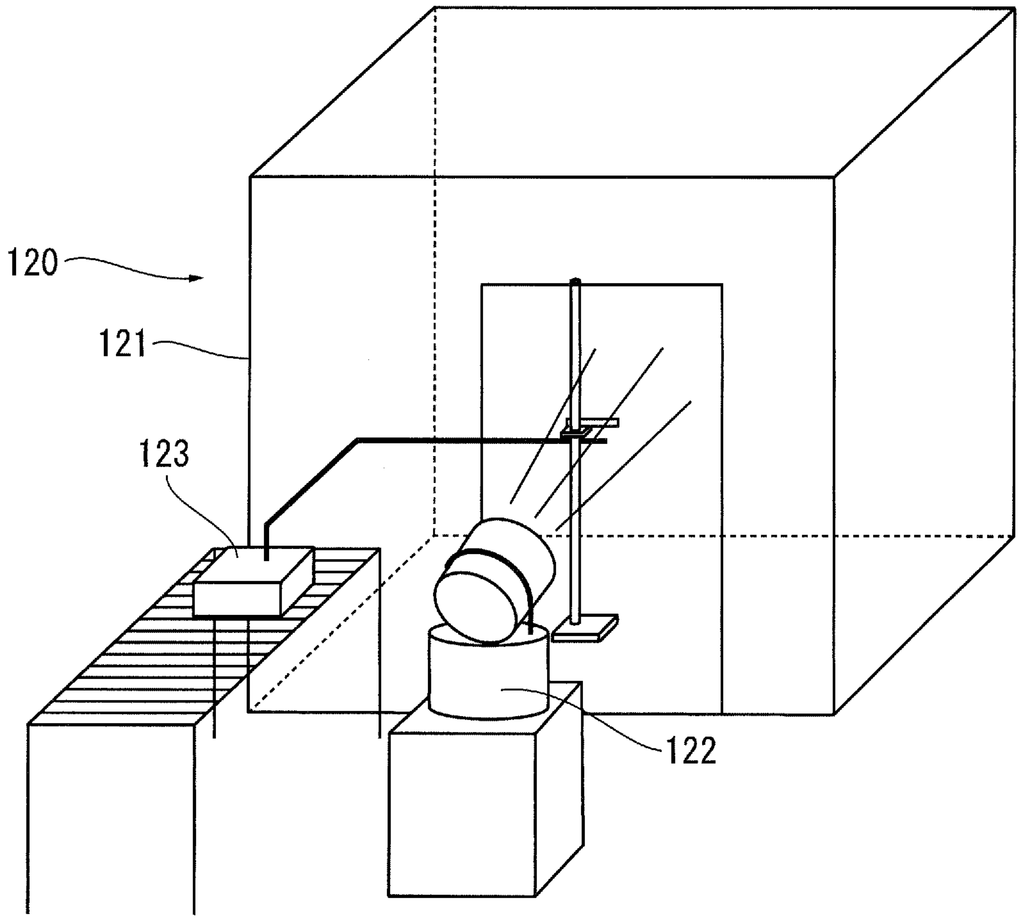

# FIG.16

## (a)SPRAY AMOUNT

| | (g) |
|---|---|
| TAP WATER | 37.5 |
| PURIFIED WATER | 39.0 |
| STABLE PURE WATER A | 33.5 |
| STABLE PURE WATER B | 33.5 |
| EXAMPLE 5 | 38.0 |

## (b)PARTICLE SIZE DISTRIBUTION OF SPRAY LIQUID

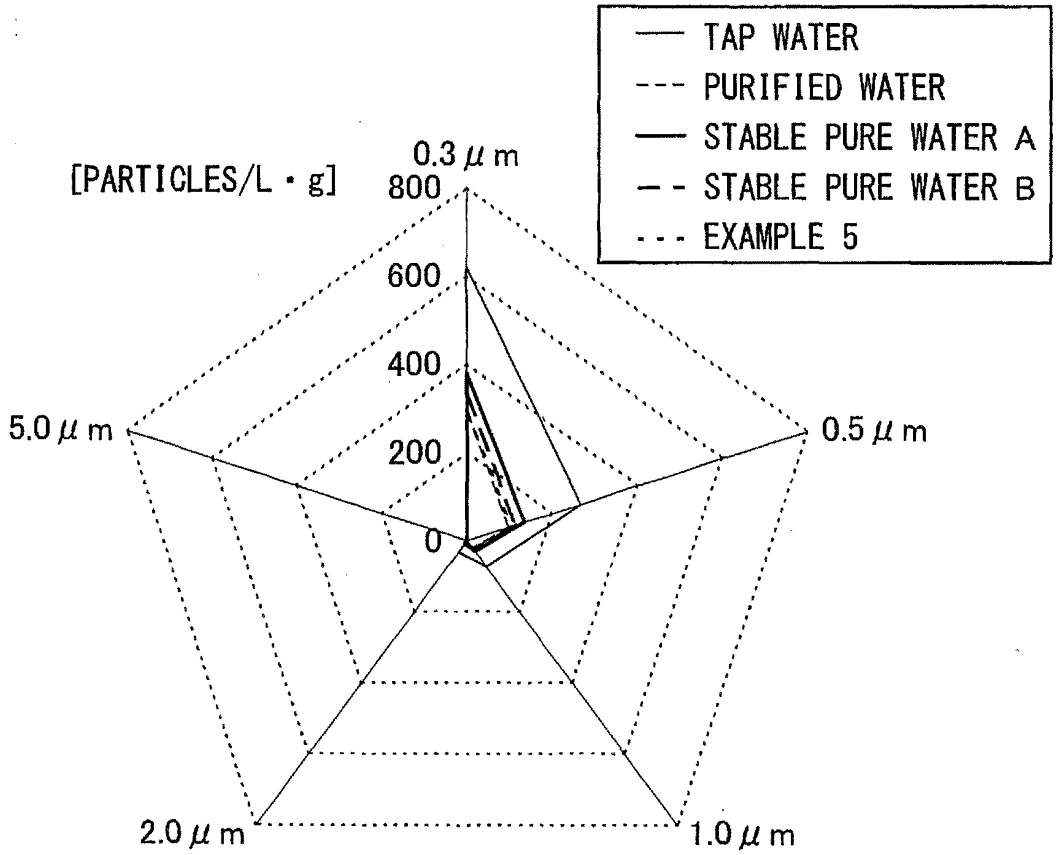

FIG.17

TIME-DEPENDENT CHANGE IN PARTICLE CONCENTRATION IN CHAMBER IN ASSOCIATION WITH SPRAYING OF TAP WATER

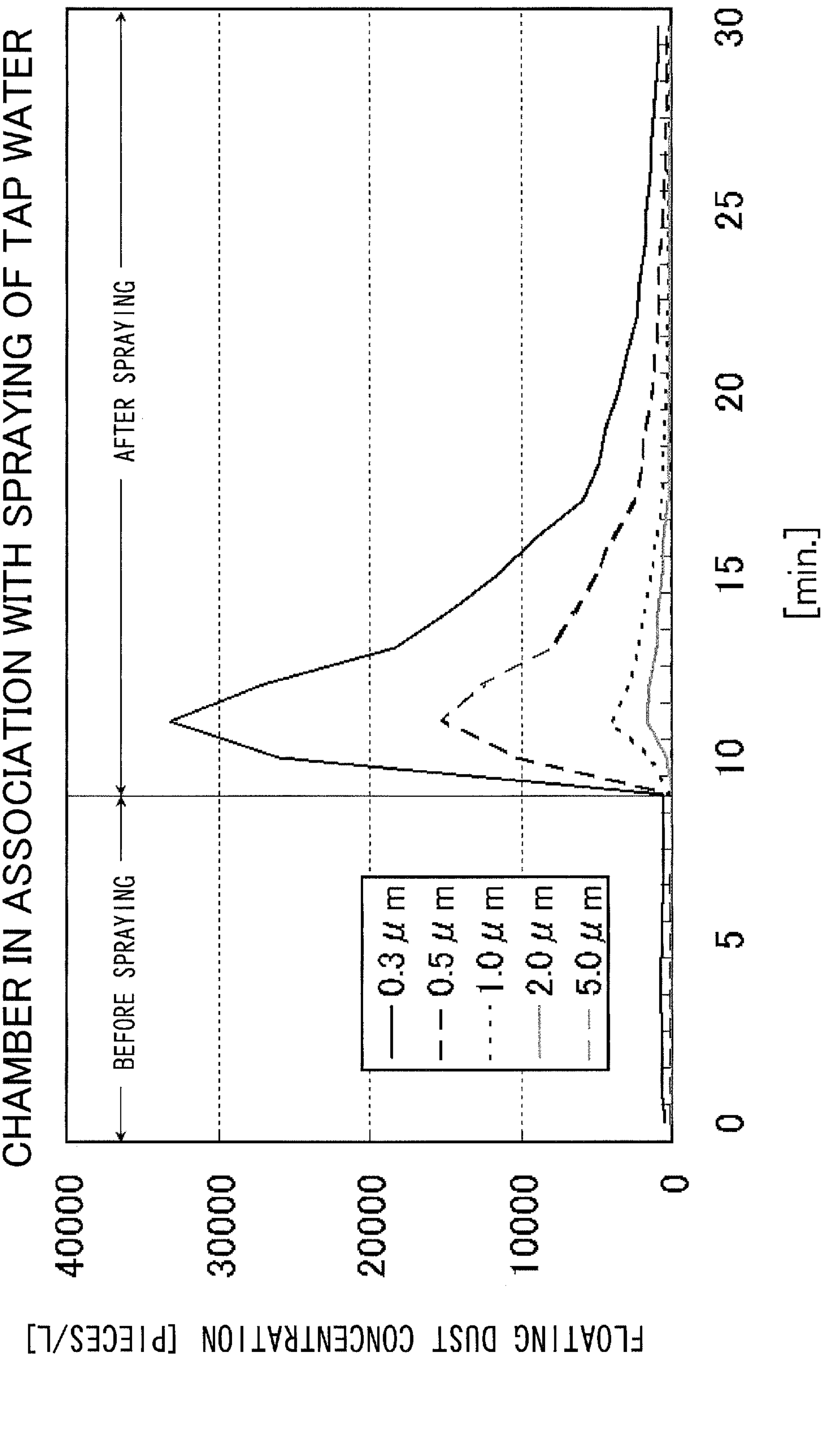

FIG.18

TIME-DEPENDENT CHANGE IN PARTICLE CONCENTRATION IN CHAMBER IN ASSOCIATION WITH SPRAYING OF PURIFIED WATER

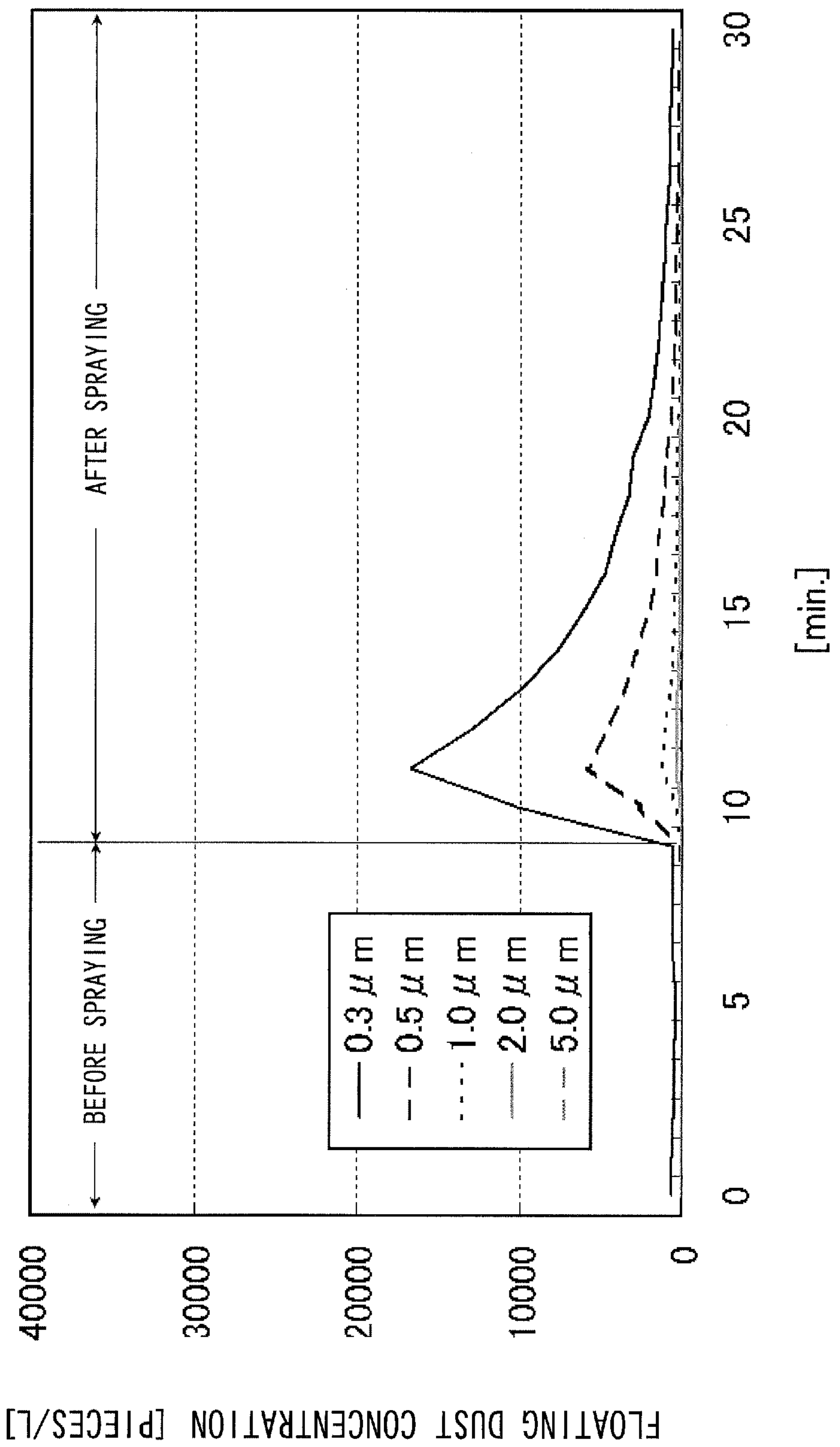

FIG.19

TIME-DEPENDENT CHANGE IN PARTICLE CONCENTRATION IN CHAMBER IN ASSOCIATION WITH SPRAYING OF STABLE PURE WATER A

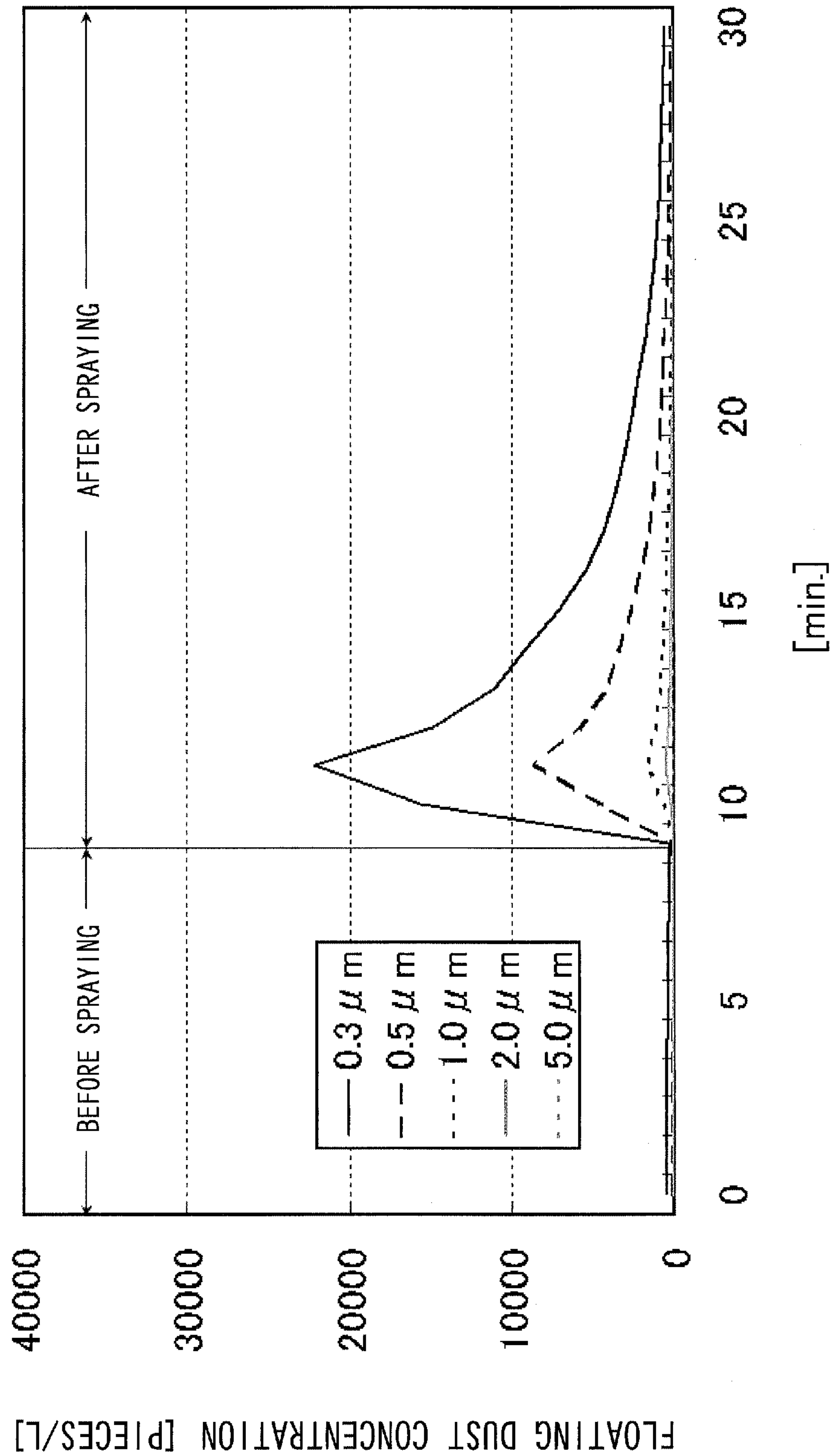

FIG.20

TIME-DEPENDENT CHANGE IN PARTICLE CONCENTRATION IN CHAMBER IN ASSOCIATION WITH SPRAYING OF STABLE PURE WATER B

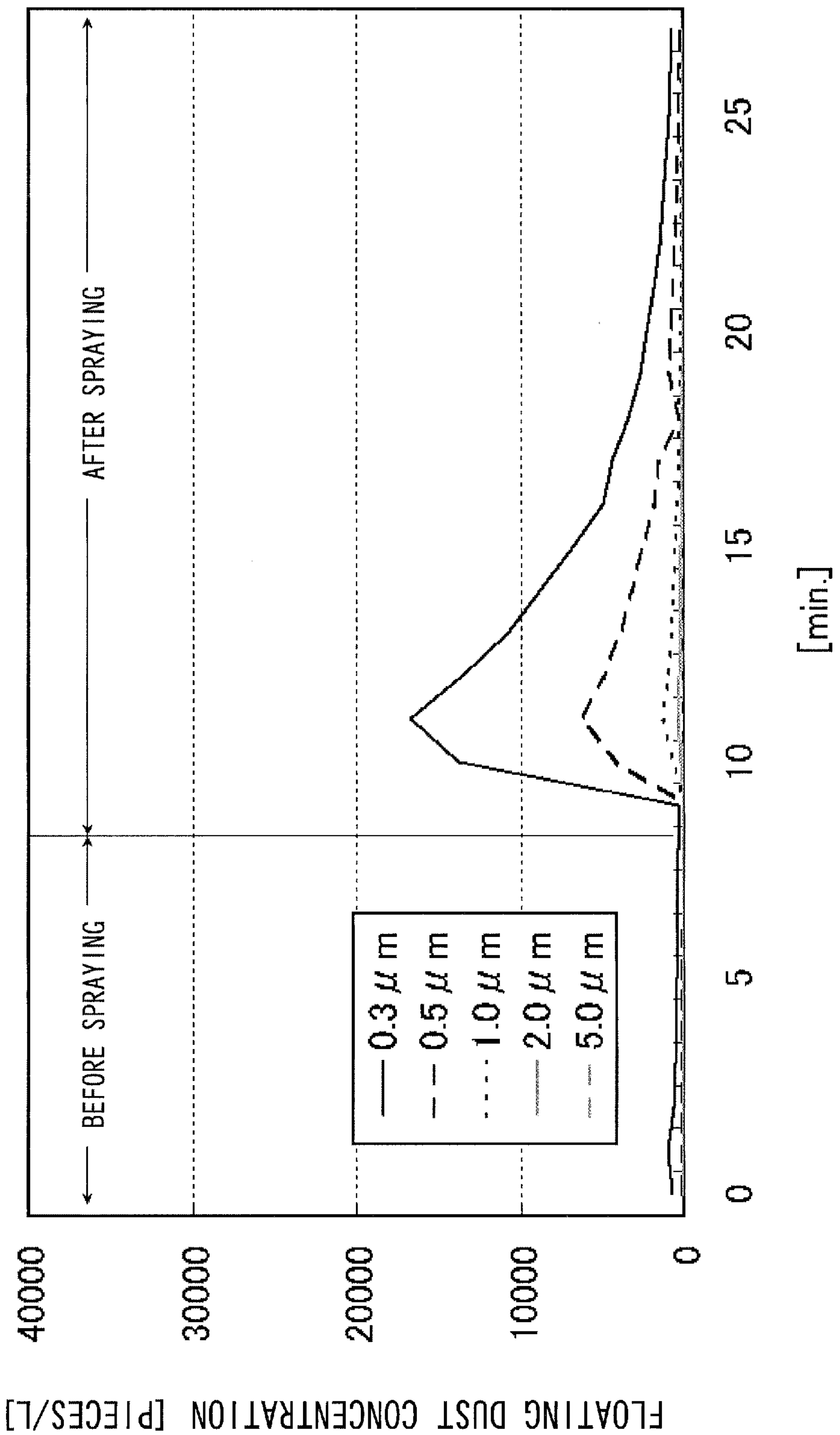

FIG.21

TIME-DEPENDENT CHANGE IN PARTICLE CONCENTRATION IN CHAMBER IN ASSOCIATION WITH SPRAYING OF EXAMPLE 5

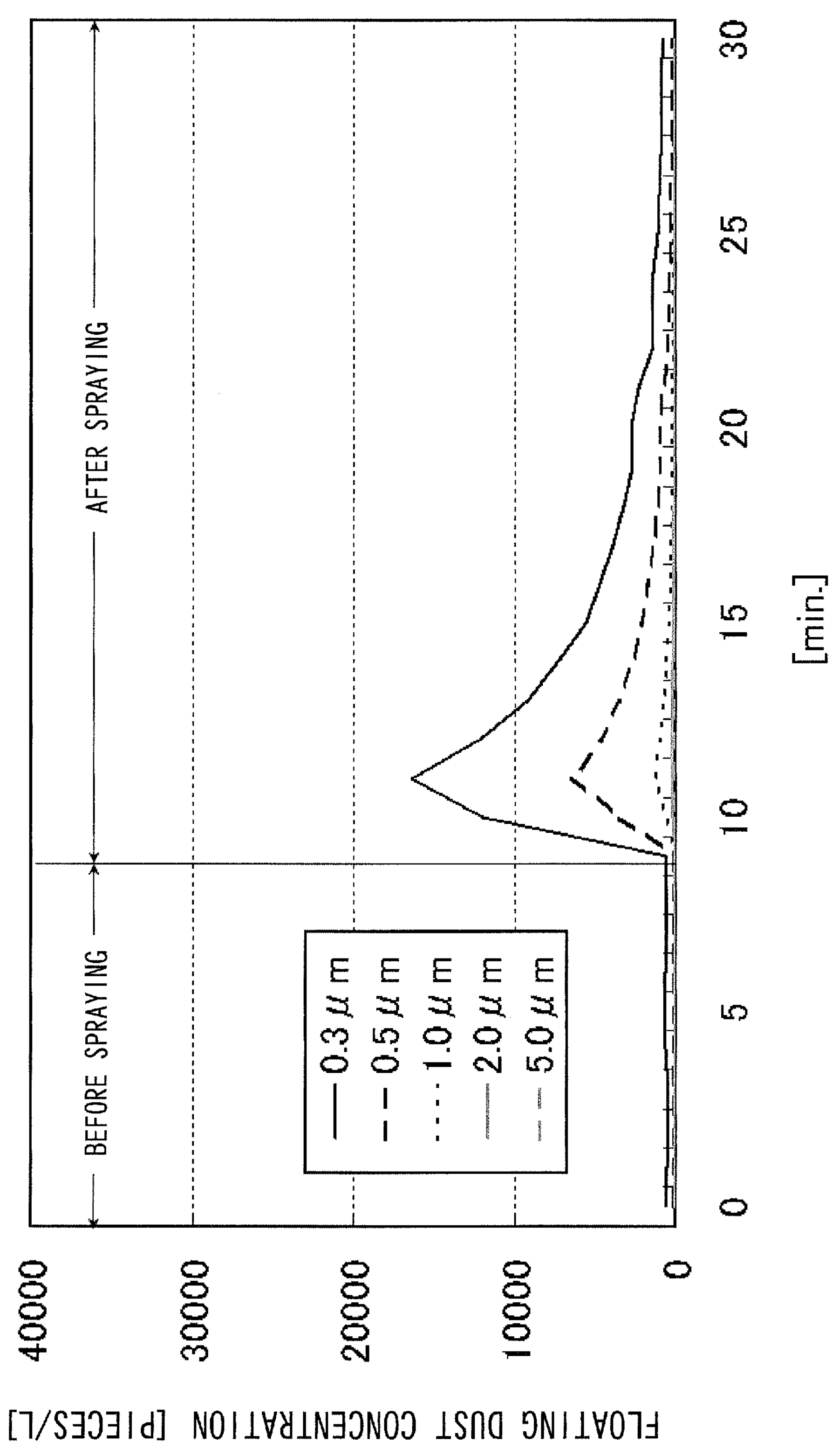

FIG.22

EXAMPLE 6

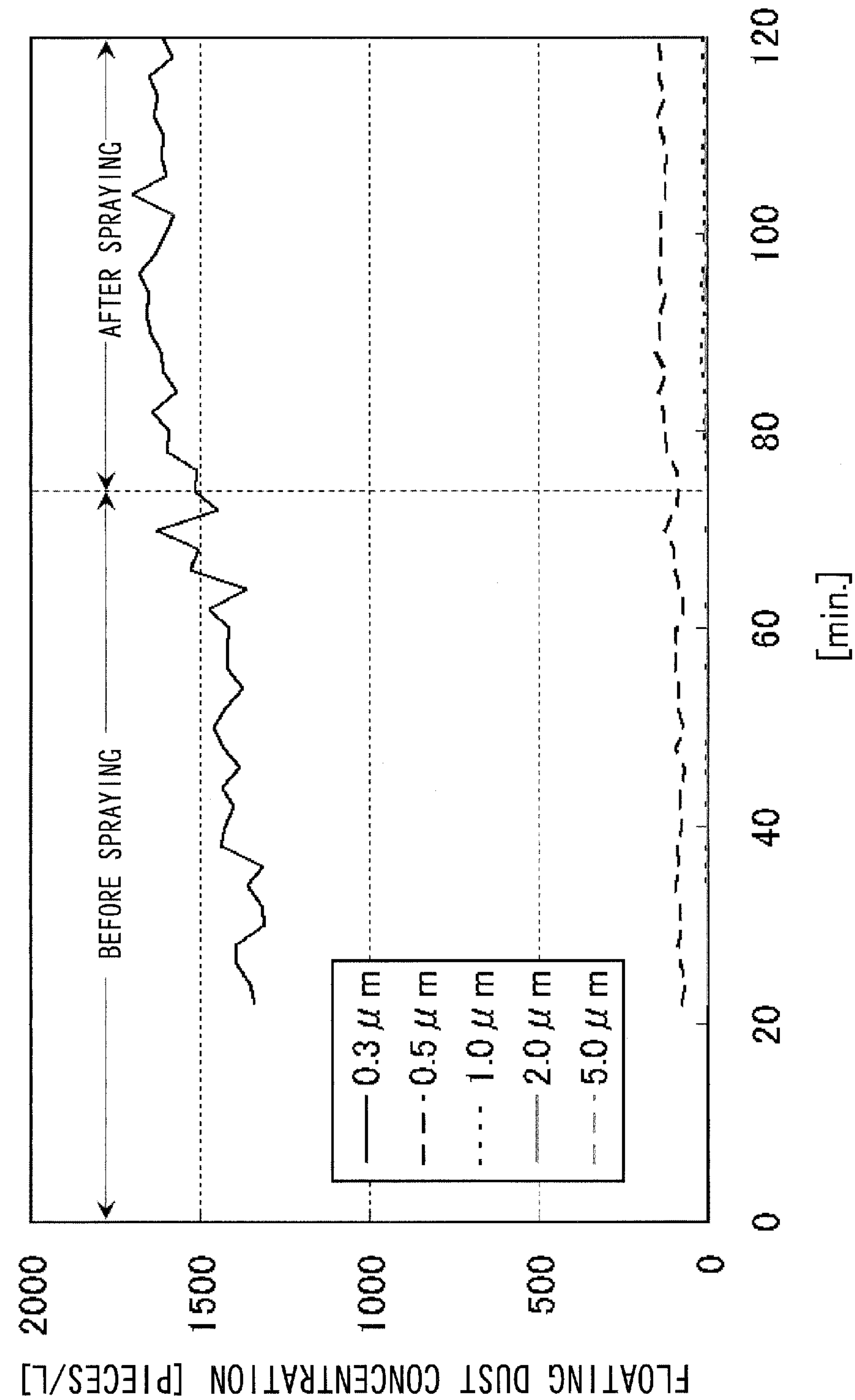

FIG.23

COMPARATIVE EXAMPLE 6-1

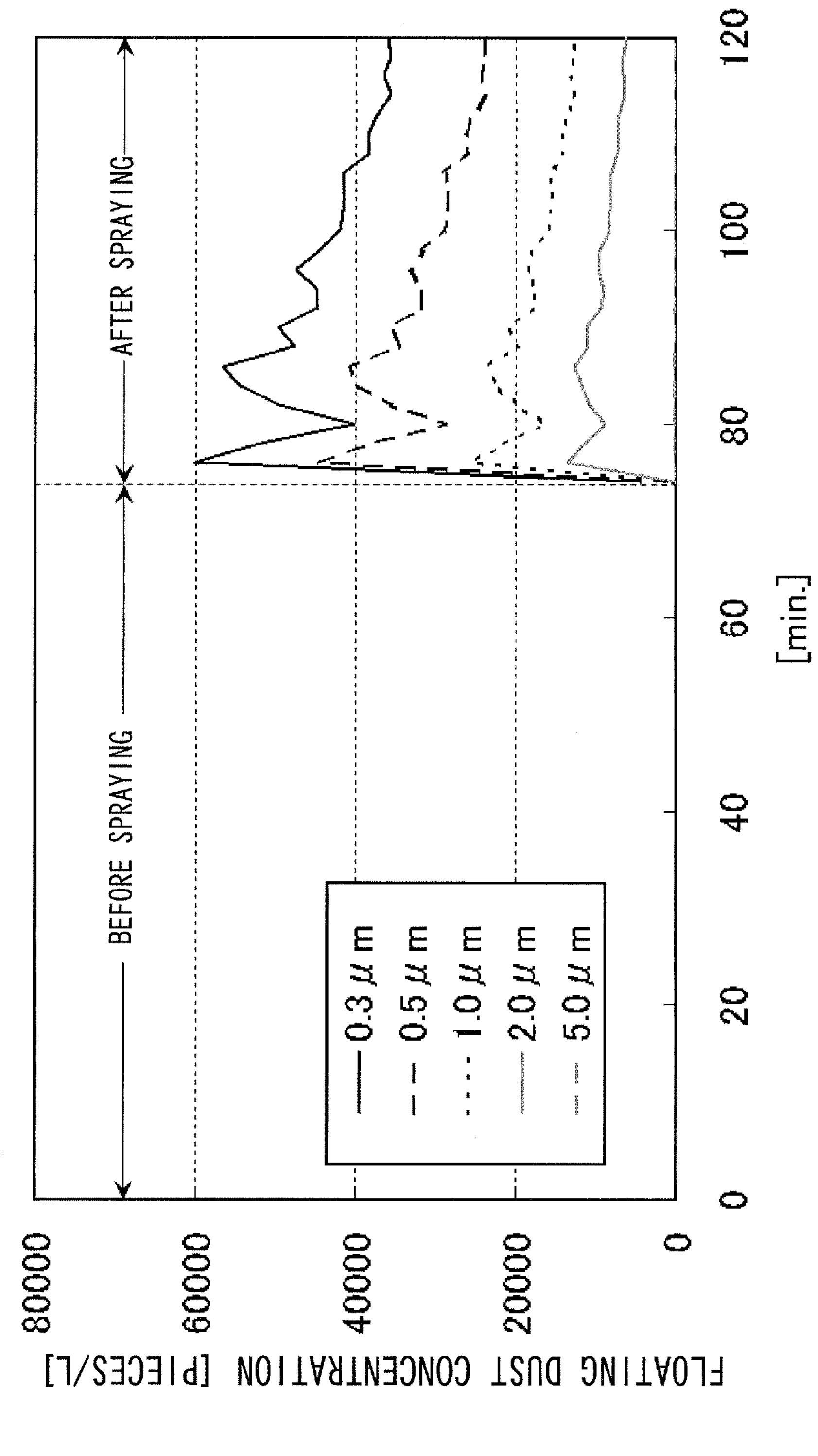

FIG.24

COMPARATIVE EXAMPLE 6-2

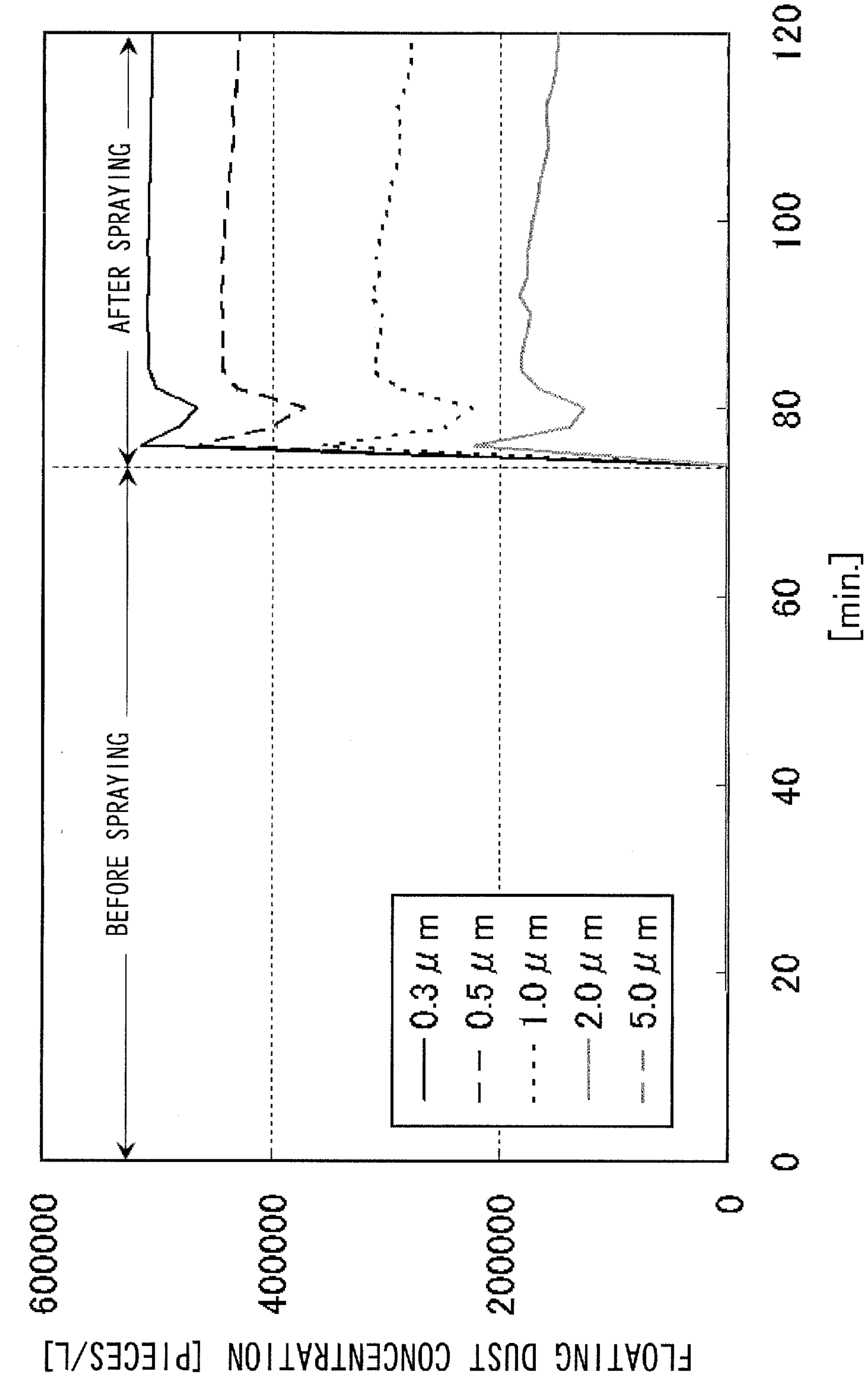

FIG.25

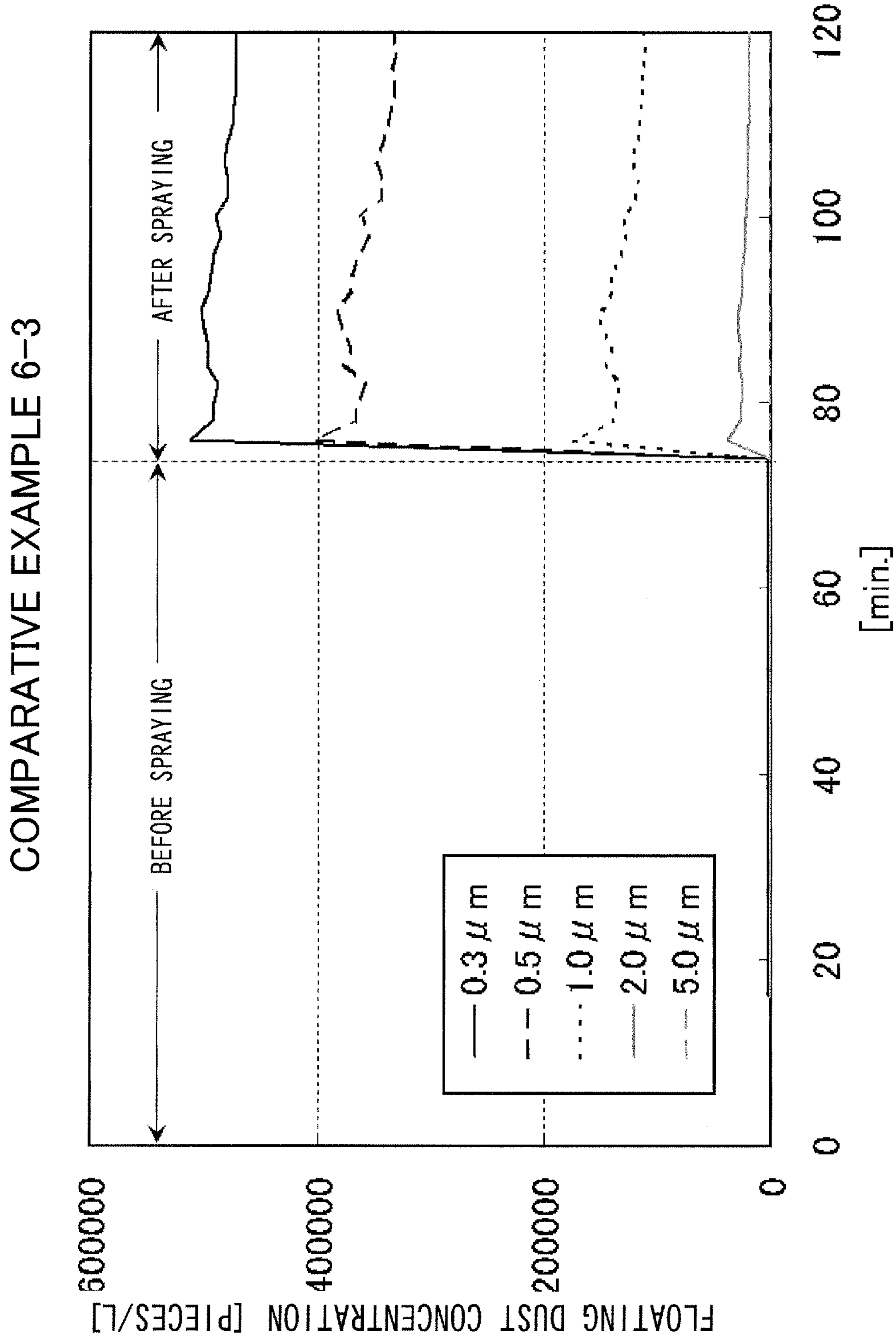
COMPARATIVE EXAMPLE 6-3
BEFORE SPRAYING
AFTER SPRAYING
FLOATING DUST CONCENTRATION [PIECES/L]
600000
400000
200000
0
0
20
40
60
80
100
120
[min.]
0.3 μm
0.5 μm
1.0 μm
2.0 μm
5.0 μm

FIG.26

COMPARATIVE EXAMPLE 6-4

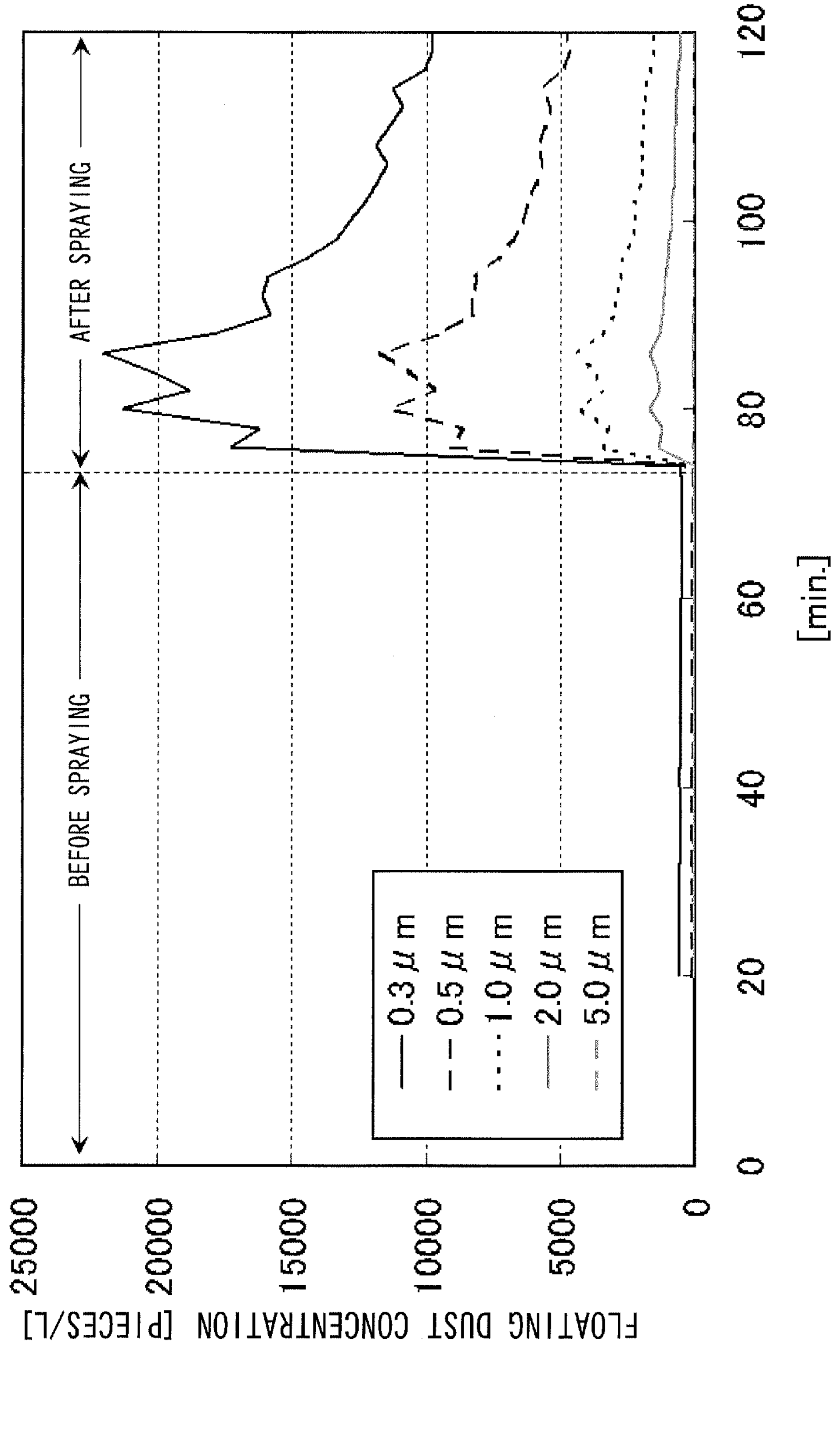

FIG.27

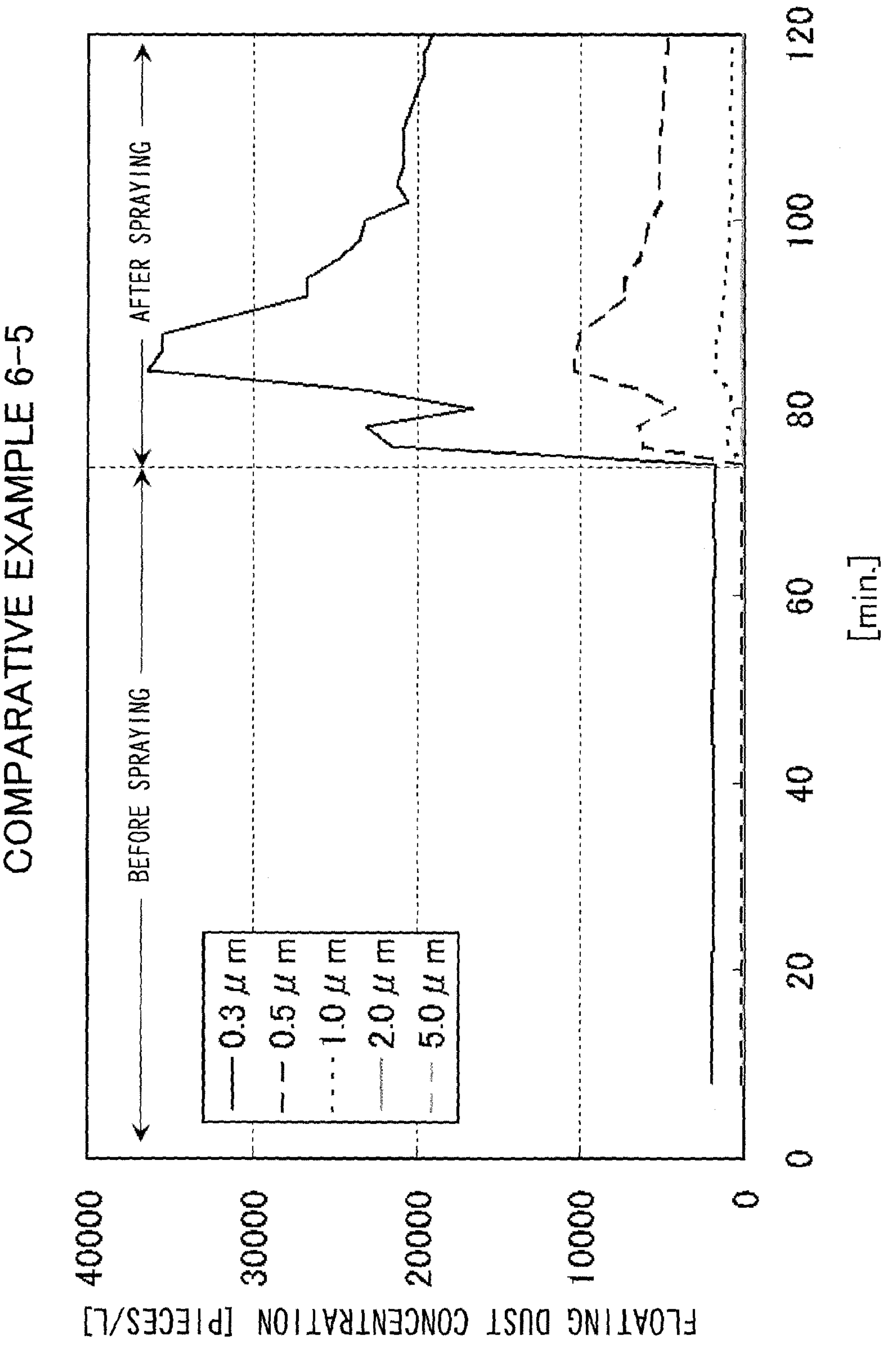
COMPARATIVE EXAMPLE 6-5
BEFORE SPRAYING
AFTER SPRAYING
0.3 μm
0.5 μm
1.0 μm
2.0 μm
5.0 μm
FLOATING DUST CONCENTRATION [PIECES/L]
40000
30000
20000
10000
0
0
20
40
60
80
100
120
[min.]

FIG.28

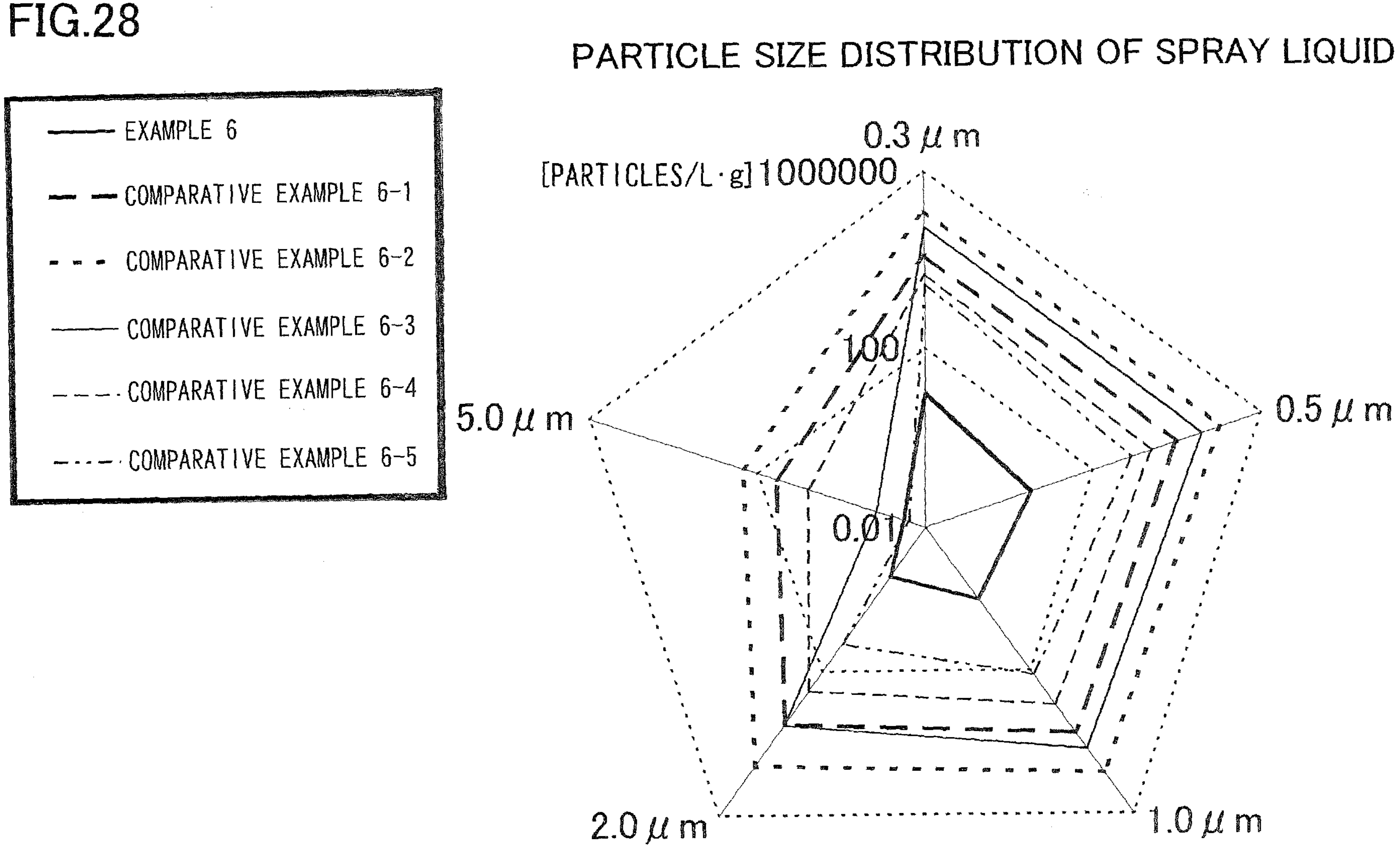
PARTICLE SIZE DISTRIBUTION OF SPRAY LIQUID
EXAMPLE 6
COMPARATIVE EXAMPLE 6-1
COMPARATIVE EXAMPLE 6-2
COMPARATIVE EXAMPLE 6-3
COMPARATIVE EXAMPLE 6-4
COMPARATIVE EXAMPLE 6-5
[PARTICLES/L·g] 1000000
100
0.01
0.3 μm
0.5 μm
1.0 μm
2.0 μm
5.0 μm

FIG.29

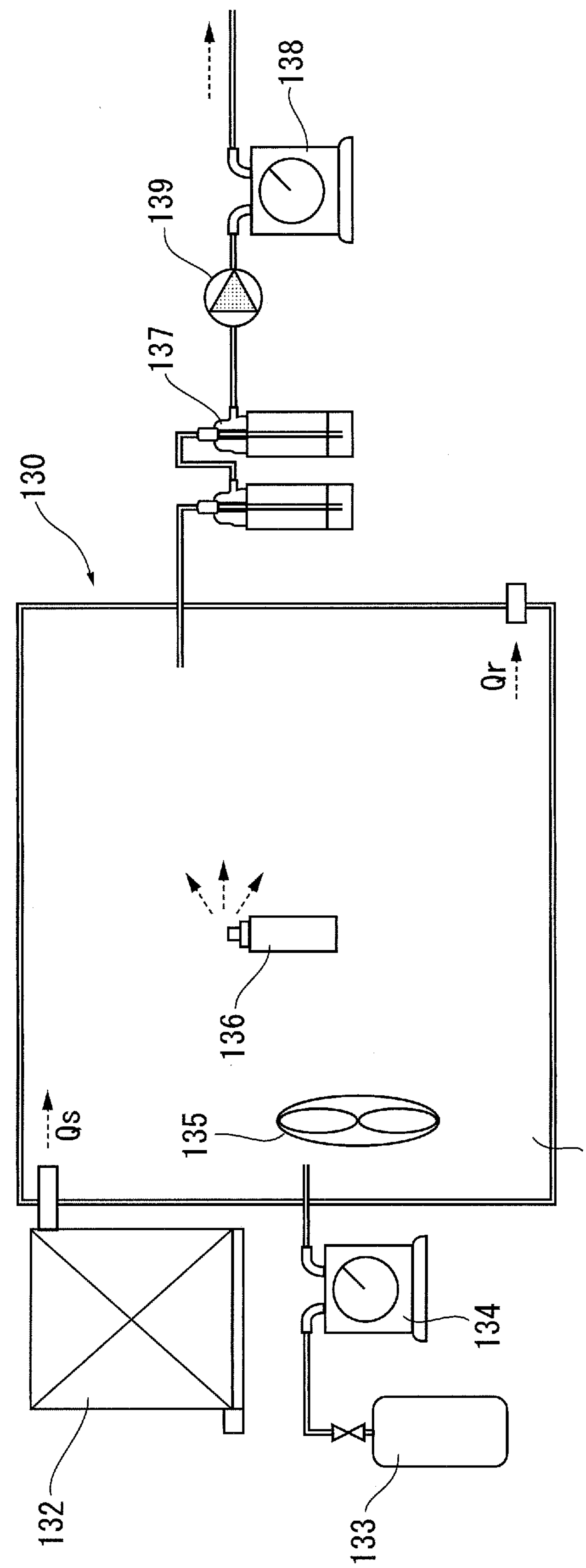

FIG.30

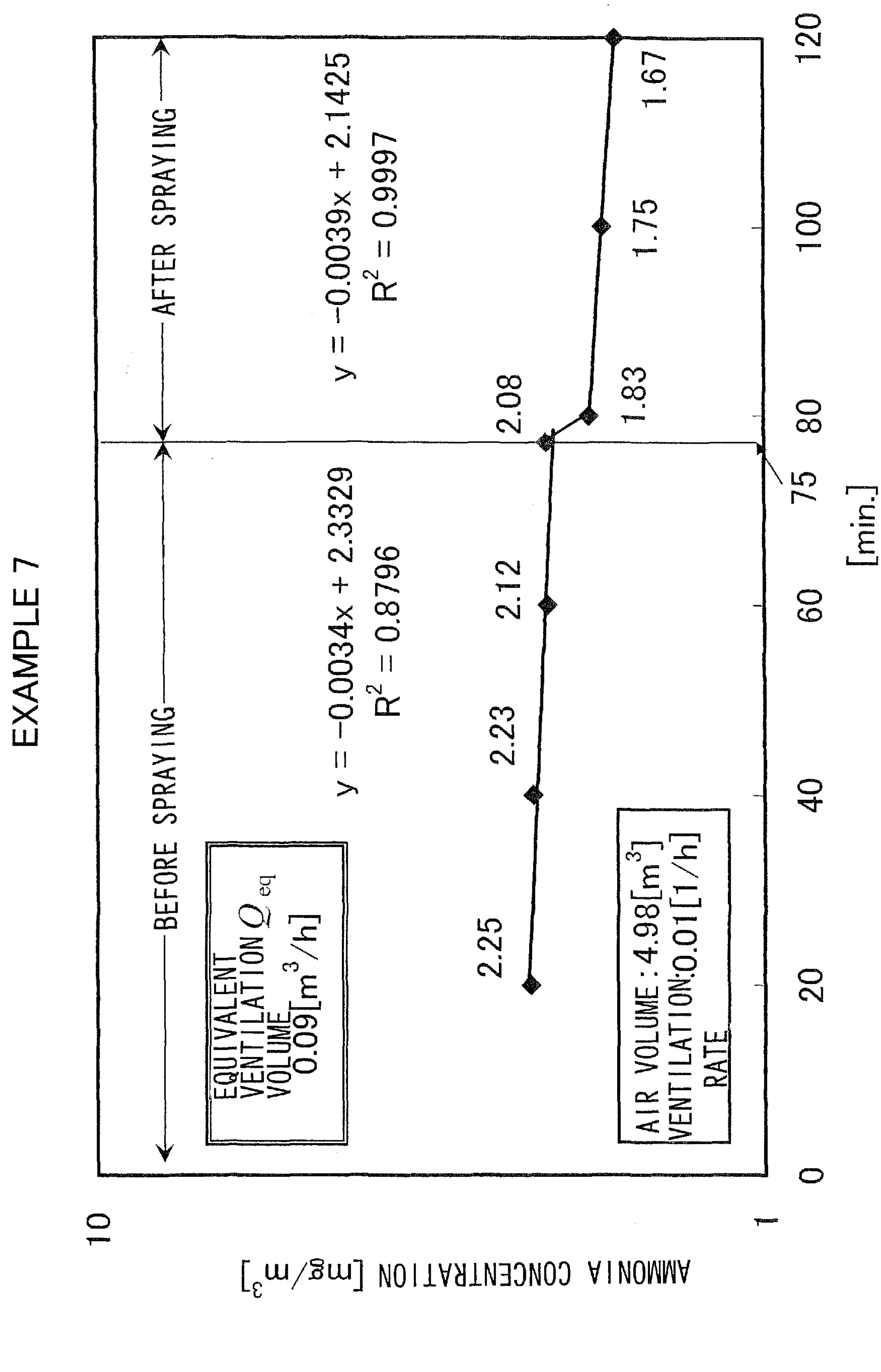
EXAMPLE 7
BEFORE SPRAYING
AFTER SPRAYING
EQUIVALENT VENTILATION VOLUME $Q_{eq}$
0.09[m$^3$/h]
y = −0.0034x + 2.3329
$R^2$ = 0.8796
y = −0.0039x + 2.1425
$R^2$ = 0.9997
2.25
2.23
2.12
2.08
1.83
1.75
1.67
AIR VOLUME : 4.98[m$^3$]
VENTILATION RATE :0.01[1/h]
10
1
0
20
40
60
75
80
100
120
[min.]
AMMONIA CONCENTRATION [mg/m$^3$]

FIG.31

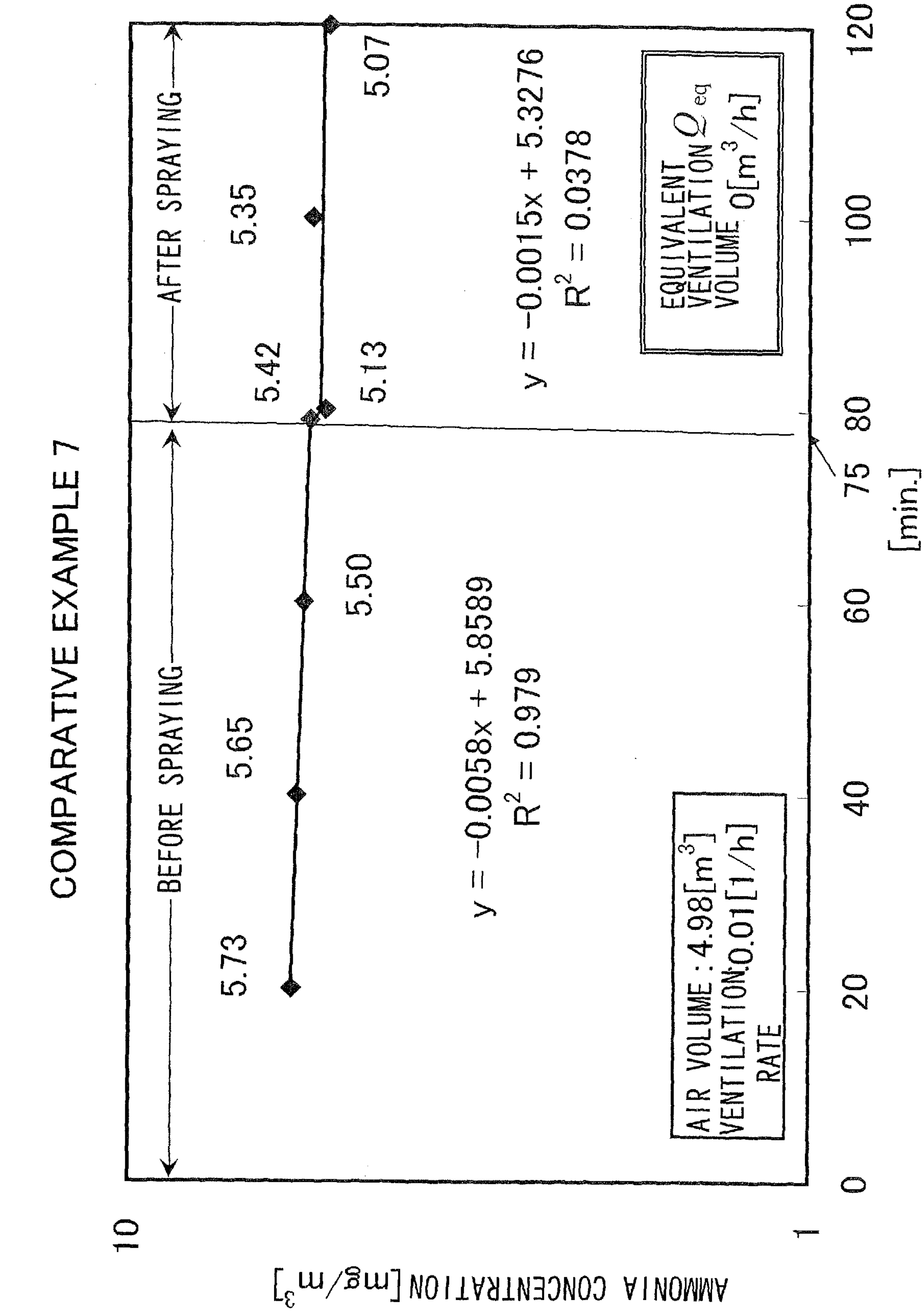
COMPARATIVE EXAMPLE 7
BEFORE SPRAYING
AFTER SPRAYING
5.73
5.65
5.50
5.42
5.13
5.35
5.07
y = −0.0058x + 5.8589
R2 = 0.979
y = −0.0015x + 5.3276
R2 = 0.0378
AIR VOLUME : 4.98[m3]
VENTILATION RATE : 0.01[1/h]
EQUIVALENT VENTILATION VOLUME Qeq 0[m3/h]
AMMONIA CONCENTRATION [mg/m3]
10
1
0
20
40
60
75
80
100
120
[min.]

**INTERNATIONAL SEARCH REPORT**

International application No.
PCT/JP2010/060333

A. CLASSIFICATION OF SUBJECT MATTER
*A61L9/01*(2006.01)i, *A61L9/14*(2006.01)i, *A62D3/30*(2007.01)i, *C02F1/30*(2006.01)i, *C02F1/68*(2006.01)i, *C09K3/00*(2006.01)i, *A62D101/20*(2007.01)n

According to International Patent Classification (IPC) or to both national classification and IPC

B. FIELDS SEARCHED

Minimum documentation searched (classification system followed by classification symbols)
A61L9/00-9/22, A62D3/30, C02F1/30, C02F1/68, C09K3/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 11-061091 A (I.H.M. Inc.), 05 March 1999 (05.03.1999), entire text (Family: none) | 1-10 |
| A | JP 2008-239632 A (Nippon Techma Engineering Corp.), 09 October 2008 (09.10.2008), entire text (Family: none) | 1-10 |
| A | JP 2004-059874 A (Terra Bio Remedic Co., Ltd., Kabushiki Kaisha Hatsu), 26 February 2004 (26.02.2004), entire text (Family: none) | 1-10 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 September, 2010 (09.09.10) | 21 September, 2010 (21.09.10) |
| Name and mailing address of the ISA/ Japanese Patent Office | Authorized officer |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3597404 B
- JP 2804823 B


### Non-patent literature cited in the description


- Common knowledge about sick house problems that you have to know. Electricity and Construction. Ohmsha, Ltd, July 2003, 101-106
- Latest knowledge for preventing sick house problems, Architectural Institute of Japan. Gihodo Shuppan Co., Ltd, March 2005, 196-208